# EUROPEAN PATENT SPECIFICATION

(11) **EP 3 395 301 B1**
(45) Date of publication and mention of the grant of the patent: **04.05.2022**
(21) Application number: 16877800.9
(22) Date of filing: 23.12.2016
(51) Int. Cl.: A61F 2/95, A61F 2/966

(54) **TENT DELIVERY SYSTEM AND ASSEMBLIES THEREOF**
STENTEINFÜHRUNGSSYSTEM UND ANORDNUNGEN DAVON
SYSTÈME DE POSE D'ENDOPROTHÈSE ET ENSEMBLES ASSOCIÉS

(30) Priority: 23.12.2015 CN 201510979117; 23.12.2015 CN 201510977057; 23.12.2015 CN 201510977060; 23.12.2015 CN 201510977059
(43) Date of publication of application: 31.10.2018
(73) Proprietor: Shanghai MicroPort Endovascular MedTech (Group) Co., Ltd., Shanghai 201318 (CN)
(72) Inventor: LI, Zhonghua, Shanghai 201318 (CN); ZHU, Qing, Shanghai 201318 (CN); PENG, Dadong, Shanghai 201318 (CN); LIU, Mengqin, Shanghai 201318 (CN); YUAN, Zhenyu, Shanghai 201318 (CN); HUANG, Dingguo, Shanghai 201318 (CN); JI, Qingru, Shanghai 201318 (CN); MIAO, Zhenghua, Shanghai 201318 (CN)
(74) Representative: Patentship Patentanwaltsgesellschaft mbH
(86) International application number: PCT/CN2016/111812
(87) International publication number: WO 2017/107990

(56) References cited:
- WO-A1-2015/164284
- WO-A2-03/068302
- CN-A- 101 933 821
- CN-A- 103 785 094
- CN-A- 104 706 449
- CN-A- 105 105 894
- CN-A- 105 943 210
- CN-A- 105 943 211
- CN-A- 105 943 212
- CN-A- 105 943 213
- US-A1- 2011 257 720
- US-A1- 2011 282 425
- US-A1- 2015 305 902

## Description

### TECHNICAL FIELD

The present invention relates, in general, to a medical surgical device and, in particular, to a stent delivery system, as well as its components.

### BACKGROUND

A stent-graft is an implantable device composed of a tubular surgical graft covering and a self-expanding framework. The stent-graft is placed inside a blood vessel to block an aneurysmal segment of the blood vessel and reestablish the blood flow so as to redirect hemodynamic pressures away from the diseased segment of the blood vessel.

In a minimally invasive interventional treatment of a blood vessel, the operating physician usually makes an incision, anatomically or paracentetically, in the wall of a small blood vessel such as the femoral artery and then advances a delivery system in which a medical implant such as a stent-graft is loaded through the incision, across the small blood vessel and other channels to a target lesion site such as the coronary artery, aortic valve, thoracic aorta, abdominal aorta, intracranial artery or carotid artery. In contrast to the medical implant (e.g., stent-graft) which is bulky and exerts a large radial force, the incision, small blood vessel and other involved channels are sized typically with a diameter of only a few millimeters, or even smaller in case of calcified blood vessels. This urgently necessitates sufficient miniaturization, i.e., outer diameter shrinkage, of the delivery system and hence of a sheathing catheter thereof in which the medical implant is housed for minimizing trauma to the patient's blood vessels and expanding the scope of indications. Meanwhile, the sheathing catheter for the interventional device must provide sufficiently high radial strength for retaining the medical implant (e.g., stent-graft) therein and sufficiently high axial strength for smooth deployment therefrom and hence safe implantation of the medical implant (e.g., stent-graft).

On the other hand, current requirements on a stent-graft delivery system include the capabilities of easy introduction, accurate, safe deployment and simple retrieval. A blood vessel with a true, false or dissecting aneurysm is in nature a damaged or diseased vessel possibly with an irregular morphology, which requires the delivery system to be well designed for smooth introduction therein. Successful implantation of the stent-graft relies on its anchoring to a healthy blood vessel segment (aneurysmal neck) which, however, tend to be limited in length. Therefore, in order to effectively block the lesion, the delivery structure is required to be able to place the stent accurately. Further, safe retrieval out of the body without causing dislocation of the anchored implant is also important.

More specifically, during the implantation, in order to prevent dislocation or eversion of the stent-graft that may occur under the impact of the blood flow, it is particularly critical to accurately position the stent graft. During the surgical procedure, it is necessary to constrain a bare stent segment at a proximal end of the stent-graft during the deployment of the main part of the stent-graft to prevent dislocation or eversion of the stent-graft. In addition, when the apposition of the proximal end to the vessel wall is not attained at an early stage of the deployment of the main body, in order for accurate placement at the target site to be achieved, fine positions adjustments of the stent-graft are required before the final deployment of the bare stent segment of the stent-graft.

Existing stent-grafts may be categorized into the following groups based on how they are deployed.
1) Stent-grafts not deployed in multiple stages. Such a stent-graft is entirely deployed as a result of the retraction of an outer sheath and is disadvantageous in that the deployed stent-graft may be dislocated under the impact of the blood flow and thus positioned inaccurately.
2) Bundled stent-grafts. A bundled stent-graft is also deployed by retracting the outer sheath in which it is loaded. Subsequently, a guidewire is operated to unravel the bundling thread to deploy the stent-graft. This design is disadvantageous in that the stent-graft may become shorter and thus shift from the target site.
3) Stent-grafts for multi-stage deployment. Bolton Medical Inc. (Bolton) filed an application, entitled "Delivery System and Method for Self-Centering a Proximal End of a Stent Graft" (Publication No. CN101415377A) and relating to a delivery system of a multi-stage deployment design in which a bare stent segment of a stent graft is inserted into securing troughs and pinned by sheet anchoring pins. After the sheet anchoring pins are removed, the bare stent segment can be deployed from the securing troughs. This design is disadvantageous in that the bare stent segment could not be stably positioned in the securing troughs, adding difficulties in insertion of the anchoring pins. Additionally, the anchoring pins are rectangular in cross section which leads to large resistance in the assembly and deployment processes. Further, the anchoring pins are moved and controlled under the actuation of coaxial catheters. However, due to friction between the coaxial catheters over a relative large area, resistance is increased in the deployment process.

US 2011/282425 A1 discloses a delivery system which comprises a handle allowing operation of the delivery system with one hand while maintaining accuracy in delivery and deployment of a prosthesis in a body lumen.

CN 105 105 894 A discloses a reinforced compound sheath for a delivery system, a preparation method and application thereof, wherein the reinforced compound sheath sequentially comprises an inner layer, a reinforced layer and an outer layer from interior to exterior, wherein the reinforced layer comprises axial reinforcing elements and a spiral reinforcing element.

WO 2015/164284 A1 discloses a delivery system for delivering a prosthesis which includes a sheath, a slide shaft having a threaded outer surface and a handle, wherein the handle includes an internal spring assembly for selectively engaging and disengaging the handle with the threaded outer surface of the slide shaft.

Furthermore, Bolton also filed an application, entitled "Stent-Graft, Graft Stent Delivery System and Kit, and Stent-Graft Implantation Method" (Application No. CN200480032425.5) and relating to a stent-graft delivery system, a graft sleeve and a multi-stage deployment system. The graft sleeve and the multi-stage deployment system, however, suffer from a lack of structural reliability, which may lead to low stent-graft deployment accuracy and tend to cause medical accidents. Further, in order for the graft sleeve and the multi-stage deployment mechanism to operate as desired, additional features for actuating them are required in a handle assembly of the delivery system. Therefore, there exists a need for a handle assembly allowing low-resistance deployment and having control features for enabling the respective deployment stages.

### SUMMARY OF THE INVENTION

The invention relates to a handle assembly as defined by claim 1 and a delivery system as defined by claim 17.

In a first aspect of the present invention, a thin-walled high-strength outer sheath of a stent delivery system is provided. The outer sheath includes an inner layer, a reinforcing layer and an outer layer which are arranged from inside outward. The reinforcing layer includes at least one first reinforcing member and at least one second reinforcing member. The first reinforcing member spirals along an axis of the outer sheath, and the second reinforcing member extends parallel to the axis of the outer sheath.

In a second aspect of the present invention, a multi-stage deployment assembly of a stent delivery system is provided, which is capable of increasing structural reliability of the delivery system, reducing stent deployment resistance, enhancing stent deployment accuracy and lowering the possibility of medical accidents. The multi-stage deployment assembly includes a conical tip, a multi-stage deployment anchor, a multi-stage deployment base, a plurality of multi-stage deployment rods and multi-stage deployment guidewires. The conical tip has a distal end defining a plurality of securing bores for receiving the respective multi-stage deployment rods. The multi-stage deployment anchor defines a plurality of guide bores allowing passage therethrough of the respective multi-stage deployment rods. The plurality of multi-stage deployment rods each have a distal end in fixed connection with the multi-stage deployment base and a proximal end that passes through a corresponding one of the guide bores in the multi-stage deployment anchor and is received within a corresponding one of the securing bores in the conical tip. The multi-stage deployment guidewires each have a proximal end in fixed connection with the multi-stage deployment base. The multi-stage deployment rods have segments between the conical tip and multi-stage deployment anchor, which are configured to be inserted through and thereby constrain a proximal end of a stent.

In a third aspect of the present invention, a handle assembly of a stent delivery system is provided, which is capable of increasing structural reliability of the delivery system, enhancing stent deployment accuracy and lowering the possibility of medical accidents. The handle assembly includes a rear handle portion, a slider and guide rods. The rear handle portion is in threaded connection with the slider and the slider is in slidable connection with the guide rods. The guide rods are disposed in parallel to an outer sheath for accommodating a stent and the slider is in axial connection with the outer sheath. The outer sheath is as defined above.

In a fourth aspect of the present invention, a stent delivery system with increased structural reliability and capable of enhancing stent deployment accuracy and lowering the possibility of medical accidents, as well as a method for using the stent delivery system, is provided. The stent delivery system includes the outer sheath as defined above, the multi-stage deployment assembly as defined above, a graft sleeve assembly and the handle assembly as defined above. The graft sleeve assembly includes a graft sleeve in which a stent is crimped and received. The stent has a proximal end constrained by the multi-stage deployment assembly and the graft sleeve is disposed within the outer sheath. The handle assembly is connected to each of the outer sheath, the graft sleeve assembly and the multi-stage deployment assembly.

The method of using the stent delivery system, which does not form part of the invention, includes the steps of:
1) introducing the stent, the graft sleeve, the outer sheath and the multi-stage deployment assembly over a delivery guidewire into a target lumen;
2) manipulating the handle assembly to advance the outer sheath axially distally, thereby deploying the graft sleeve;
3) further advancing the stent, the graft sleeve and the multi-stage deployment assembly over the delivery guidewire to a target site in the target lumen;
4) manipulating the handle assembly to control the graft sleeve assembly so that the graft sleeve therein advances axially distally, resulting in initial deployment of the stent in the graft sleeve with the proximal end of the stent still being constrained by the multi-stage deployment assembly;
5) manipulating the handle assembly to control the multi-stage deployment assembly so that the proximal end of the stent is deployed, accomplishing final deployment of the stent; and
6) retracting the graft sleeve, the outer sheath and the multi-stage deployment assembly over the delivery guidewire.

Compared to the prior art, the present invention offers the following advantages:
The stent delivery system provided in the invention is capable of more reliable deployment by virtue of the design of the outer sheath, the multi-stage deployment assembly, the graft sleeve assembly and the handle assembly and the design of the connections between these components. The method proposed in the invention for using the delivery system involves, sequentially, deployment of the graft sleeve, initial deployment of the stent and final deployment of the stent, accomplished respectively by the outer sheath, the graft sleeve assembly and the multi-stage deployment assembly under the control of the handle assembly. This method, in combination with the overall design of the delivery system, allows increased stent deployment accuracy of the delivery system and reduced possibility of medical accidents.

Additionally, in the outer sheath of the stent delivery system, the reinforcing layer including at least one first reinforcing member and at least one second reinforcing member is disposed between the inner and outer layers. The first reinforcing member spirals along the axial of the outer sheath, thereby imparting desired radial strength to the outer sheath. Meanwhile, the second reinforcing member extends in parallel to the axis of the outer sheath, thereby increasing the radial strength of the outer sheath while imparting desired axial strength thereto. As such, the overall strength of the outer sheath is enhanced at the expense of an insignificant rise in its wall thickness. In other words, the outer sheath is a thin-walled high-strength sheath. Moreover, as the first reinforcing member is interwoven with, but not disposed separate from, the second reinforcing member, the inner and outer layers of the outer sheath are free of bumps which may lead to additional friction between the interventional device and the blood vessel wall and between the inner or outer layer of the outer sheath and the blood vessel wall and additional trauma to the blood vessel. Additionally, this can also avoid limited bendability of the second reinforcing member occurring when it is fixed to the inner or outer layer of the outer sheath.

Furthermore, the multi-stage deployment assembly in the stent delivery system is able to constrain the proximal end of the stent by the multi-stage deployment rods. In order to load the stent, the multi-stage deployment rods are inserted through the proximal end of the stent and pushed by the multi-stage deployment base into the securing bores in the conical tip so that the stent is constrained at the proximal end. In order to deployment the proximal end of the stent, the multi-stage deployment guidewires are pulled back, causing axial retraction of the multi-stage deployment base and hence of the multi-stage deployment rods fixed thereto. When the multi-stage deployment rods dislodge from the securing bores in the conical tip, the proximal end of the stent disengage from the multi-stage deployment rods and is deployed. With the aid of the multi-stage deployment anchor, the multi-stage deployment assembly can effectively retain the proximal end of the stent and allows the multi-stage deployment rods to axially move in a more reliable way. In addition, as the contact area between the multi-stage deployment rods and the securing bores is small, the deployment is subject to small resistance and can be performed with higher accuracy.

### BRIEF DESCRIPTION OF THE DRAWINGS

The present invention will be further explained with reference to the accompanying drawings, in which:
Fig. 1 is a structural schematic of a stent delivery system in accordance with an embodiment of the present invention;
Fig. 2 is an enlarged schematic view of the interior of an outer sheath in the stent delivery system in accordance with an embodiment of the present invention;
Fig. 3 is a structural schematic of an outer sheath in the stent delivery system in accordance with an embodiment of the present invention;
Fig. 4 is a structural schematic of a multi-stage deployment assembly in the stent delivery system in accordance with an embodiment of the present invention;
Fig. 5 is a structural schematic of a multi-stage deployment base in the stent delivery system in accordance with an embodiment of the present invention;
Fig. 6 is a structural schematic of a graft sleeve assembly in the stent delivery system in accordance with an embodiment of the present invention;
Fig. 7 is a structural schematic of a rotary retraction mechanism in the stent delivery system in accordance with an embodiment of the present invention;
Fig. 8 is a disassembled view of an anti-reverse rotation fastener and an outer sheath in the stent delivery system, which are to be assembled in a first manner, in accordance with an embodiment of the present invention;
Fig. 9 shows the anti-reverse rotation fastener and the outer sheath in the stent delivery system, which are assembled together in the first manner, in accordance with an embodiment of the present invention;
Fig. 10 is a disassembled view of an anti-reverse rotation fastener and an outer sheath the stent delivery system, which are to be assembled in a second manner, in accordance with an embodiment of the present invention;
Fig. 11 shows the anti-reverse rotation fastener and the outer sheath in the stent delivery system, which are assembled together in the second manner, in accordance with an embodiment of the present invention;
Fig. 12 is a structural schematic of a deployment structure in the stent delivery system in accordance with an embodiment of the present invention;
Fig. 13 is an assembled plan view of a control disk in the stent delivery system in accordance with an embodiment of the present invention;
Fig. 14 is a cross-sectional view of a deployment structure in the stent delivery system in accordance with an embodiment of the present invention;
Fig. 15 is a structural schematic of a left handle knob half in the stent delivery system in accordance with an embodiment of the present invention;
Fig. 16 is an assembled plan view of a handle knob in the stent delivery system in accordance with an embodiment of the present invention;
Fig. 17 is a structural schematic of a control disk in the stent delivery system in accordance with an embodiment of the present invention;
Fig. 18 is a structural schematic of a handle knob in the stent delivery system in accordance with an embodiment of the present invention;
Fig. 19 schematically shows a first step of a method for implanting a stent using the stent delivery system in accordance with an embodiment of the present invention;
Fig. 20 schematically shows a second step of the method for implanting a stent using the stent delivery system in accordance with an embodiment of the present invention;
Fig. 21 schematically shows a third step of the method for implanting a stent using the stent delivery system in accordance with an embodiment of the present invention;
Fig. 22 schematically shows a fourth step of the method for implanting a stent using the stent delivery system in accordance with an embodiment of the present invention;
Fig. 23 schematically shows a fifth step of the method for implanting a stent using the stent delivery system in accordance with an embodiment of the present invention; and
Fig. 24 schematically shows a sixth step of the method for implanting a stent using the stent delivery system in accordance with an embodiment of the present invention.

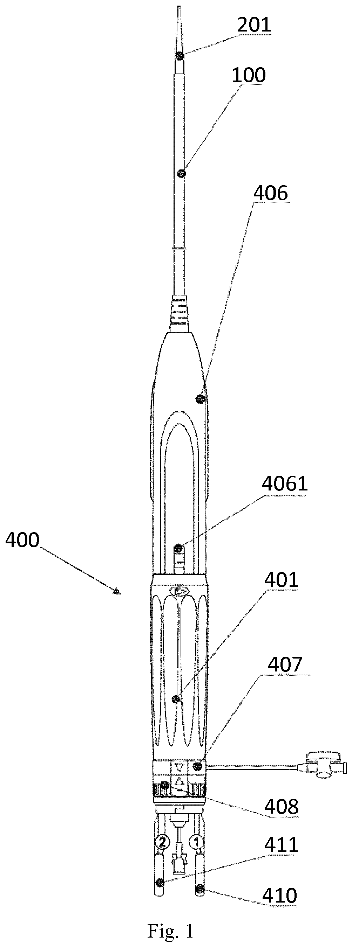

In Figs. 1 to 24, 1-inner tube; 2-stent; 21-proximal bare segment of a stent; 100-outer sheath; 101-inner layer; 102-reinforcing layer; 1021-first reinforcing member; 1022-second reinforcing member; 103-outer layer; 200-multi-stage deployment assembly; 201-conical tip; 2011-securing bore; 202-multi-stage deployment anchor; 2021-guide bore; 203-multi-stage deployment base; 2031-channel; 2032-first bore; 2033-second bore; 204-multi-stage deployment rod; 205-multi-stage deployment guidewire; 206-limiting block; 300-graft sleeve assembly; 301-graft sleeve; 302-graft sleeve connecting tube; 303-graft deployment guidewire; 400-handle assembly; 401-rear handle portion; 402-outer sheath connector; 4021-I-shaped protrusion; 4022-protrusion; 403-slider; 404-guide rod; 4041-one-way tooth slot; 405-anti-reverse rotation fastener; 4051-anti-reverse rotation elastic blade; 4052-connecting feature; 4053-notch; 4054-L-shaped notch; 406-front handle portion; 4061-locking feature; 407-handle knob; 4071-semicircular groove; 4072-left handle knob half; 4073-right handle knob half; 4074-bore; 4075-assembly pin; 4076-gap; 4077-one-way elastic blade; 408-control disk; 4081-deployment notch; 4082-one-way tooth; 409-end cover; 410-first lug; 4101-lug shaft; 4102-limiting protrusion; 4103-lug handle; 4104-limiting member; 411-second lug; 4111-lug shaft; 4112-limiting protrusion; 4113-lug handle; 4114-limiting member.

### DETAILED DESCRIPTION

A stent delivery system proposed in the present invention will be described in greater detail with reference to specific embodiments and the accompanying drawings. Features and advantages of the invention will be more apparent from the following detailed description, and from the appended claims. It is noted that the figures are provided in a very simplified form not necessarily presented to scale, with the only intention of facilitating convenience and clarity in explaining the embodiments.

The core idea of the present invention is to provide a stent delivery system capable of more reliable deployment by virtue of the design of an outer sheath, a multi-stage deployment assembly, a graft sleeve assembly and a handle assembly, as its components, and of connections between these components. A method proposed in this disclosure, not forming part of the invention, for using the above delivery system involves, sequentially, deployment of the graft sleeve, initial deployment of the stent and final deployment of the stent, accomplished respectively by the outer sheath, the graft sleeve assembly and the multi-stage deployment assembly under the control of the handle assembly. This method, in combination with the overall design of the delivery system, allows increased stent deployment accuracy of the delivery system and reduced possibility of medical accidents.

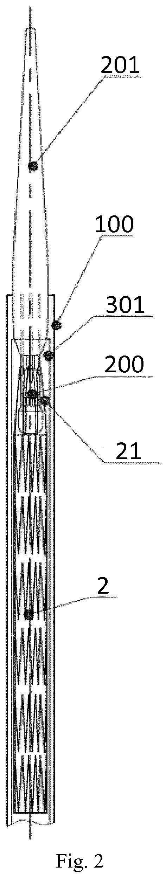

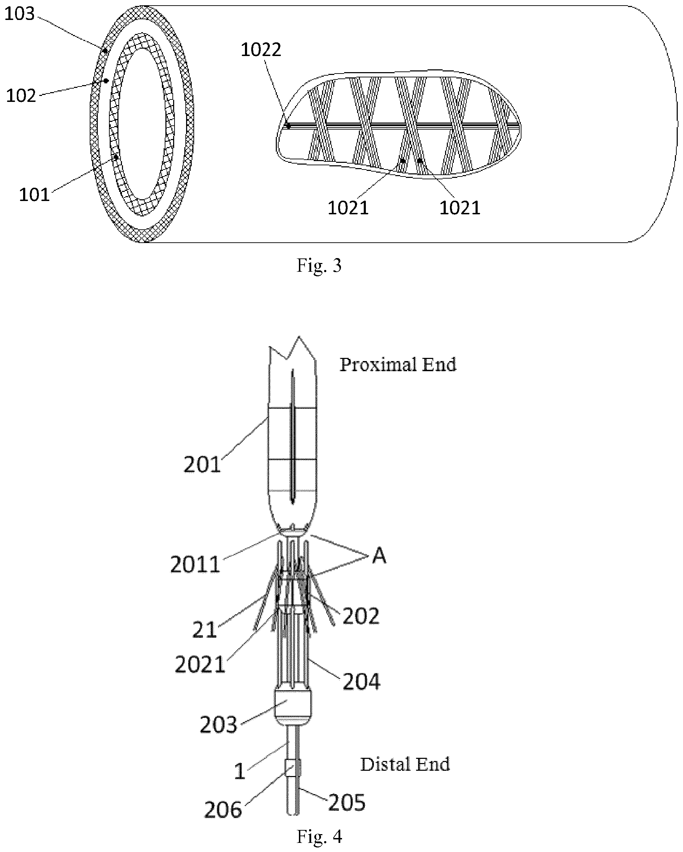

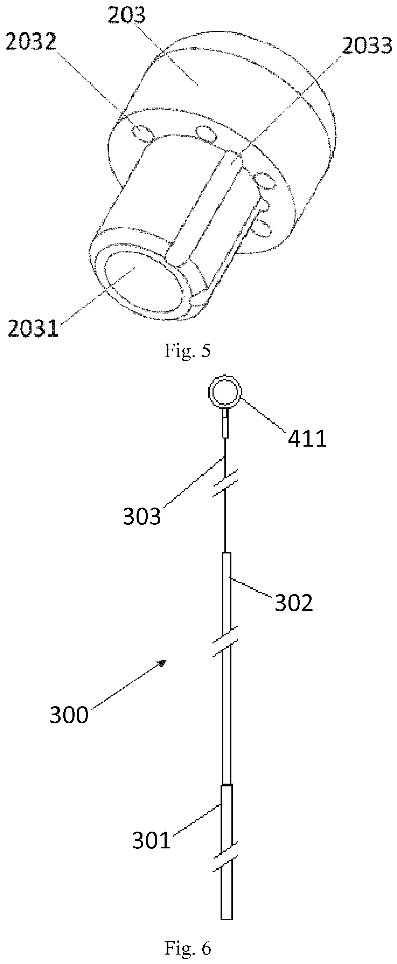

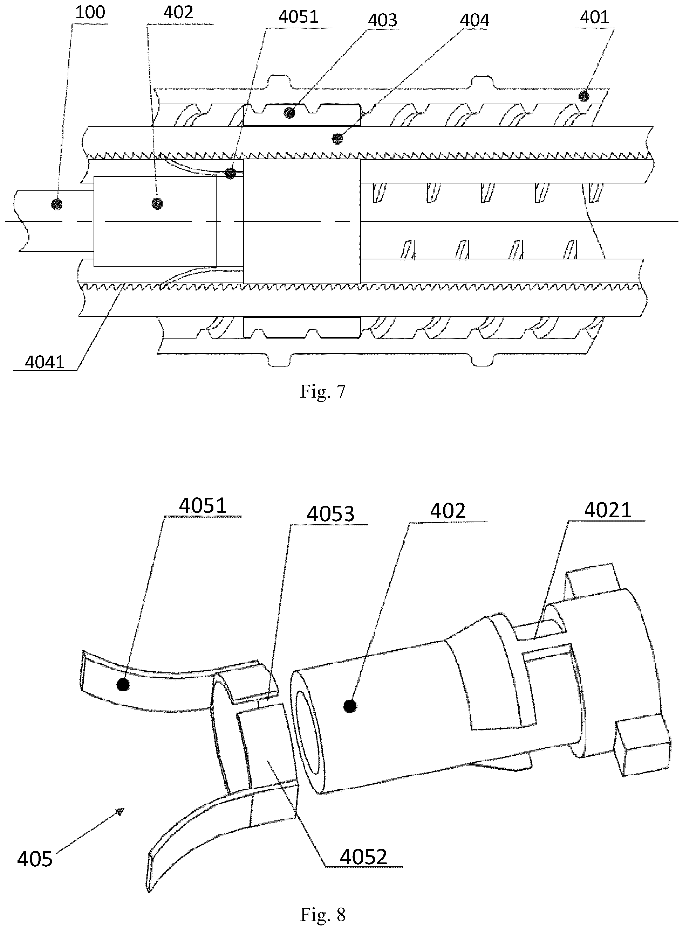

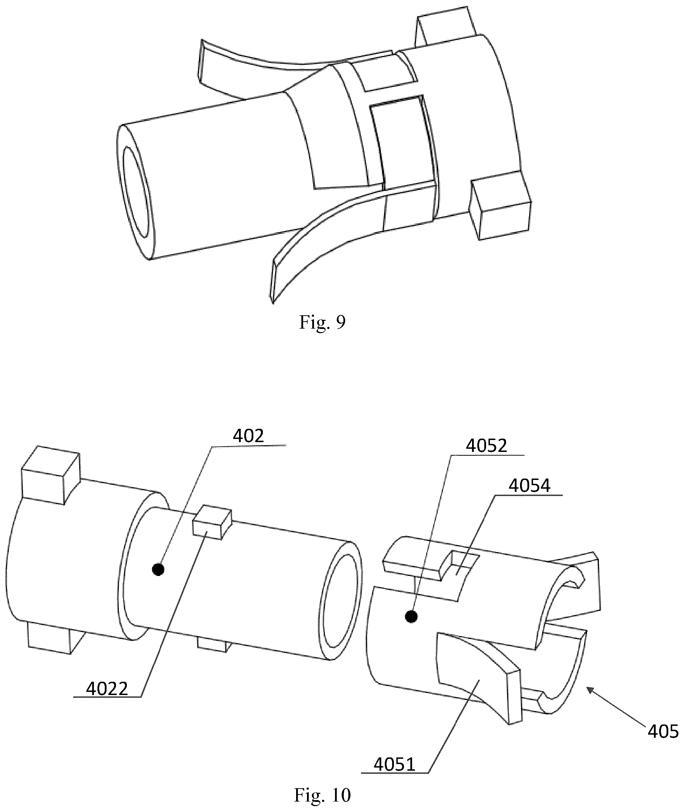

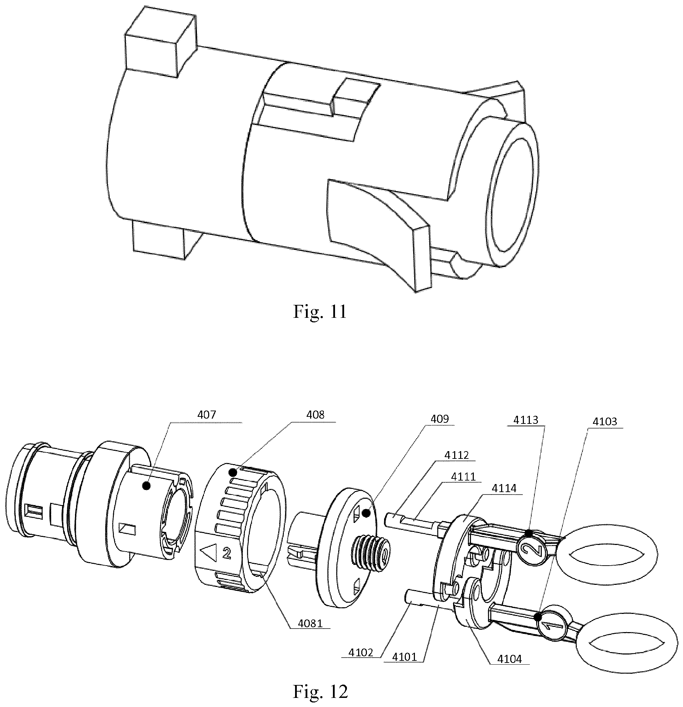

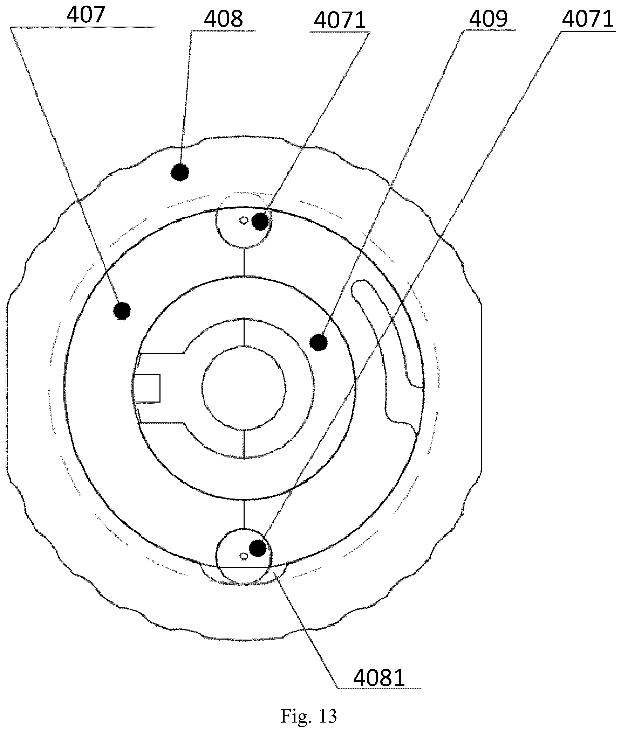

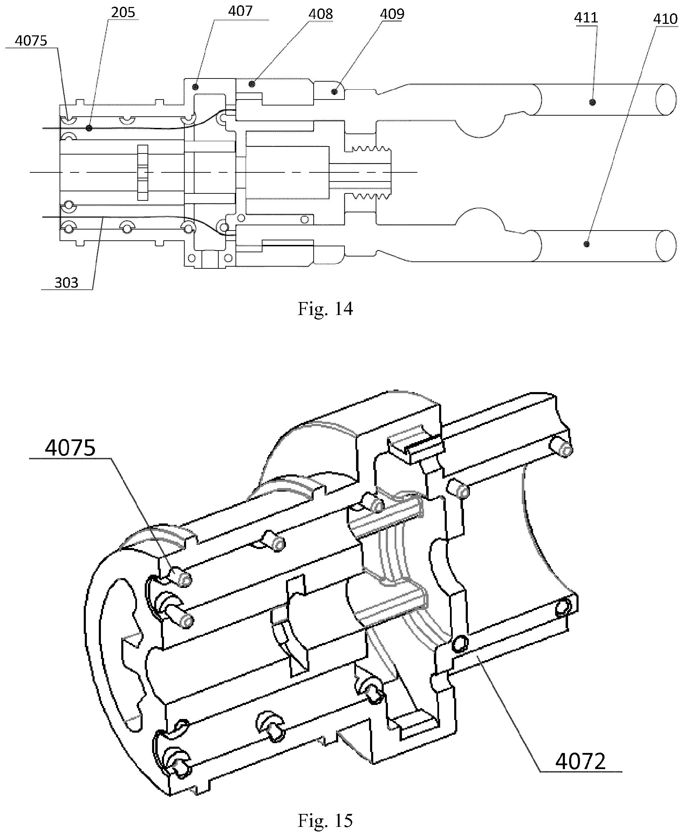

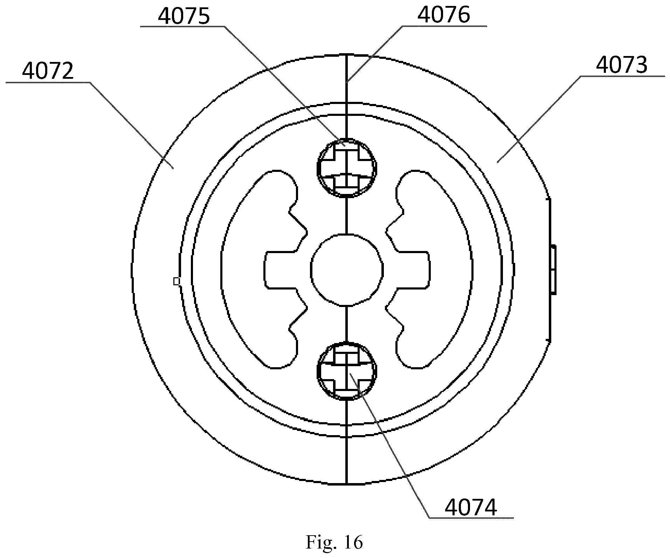

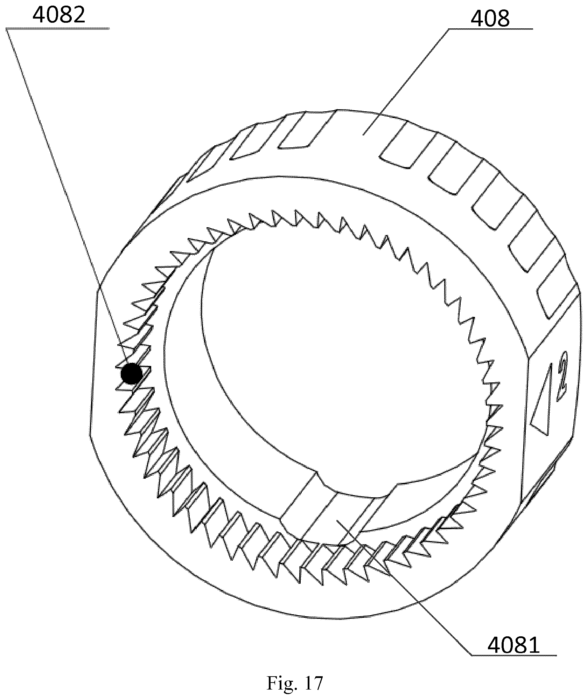

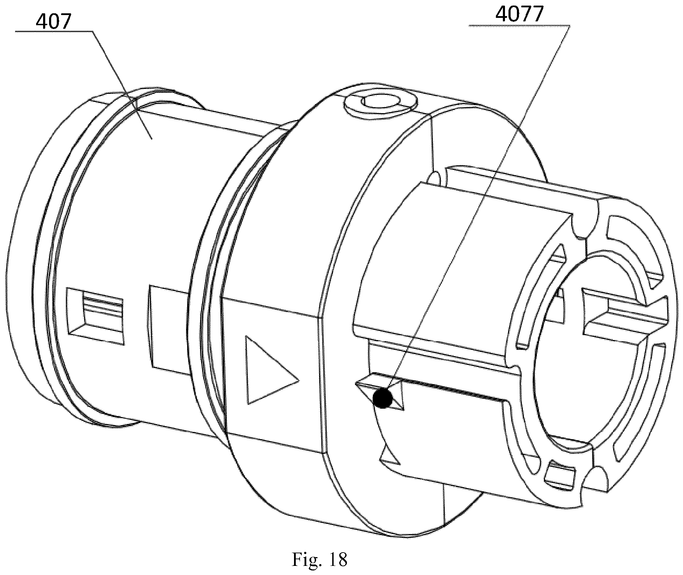

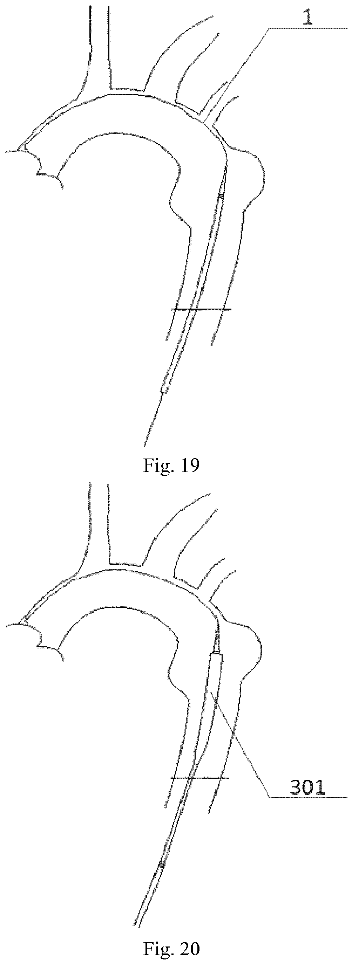

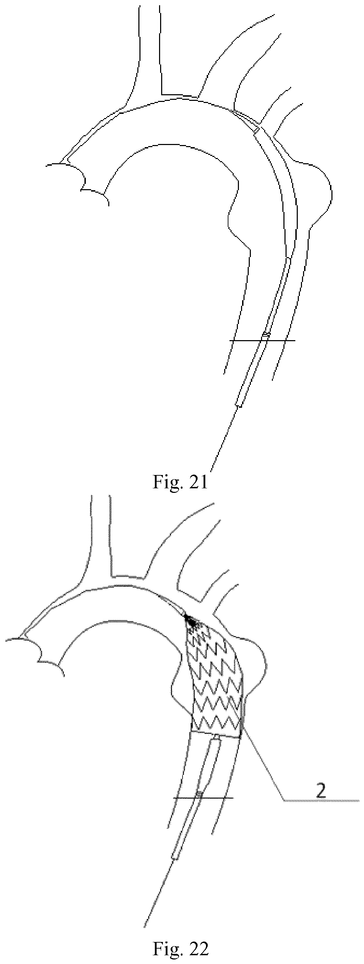

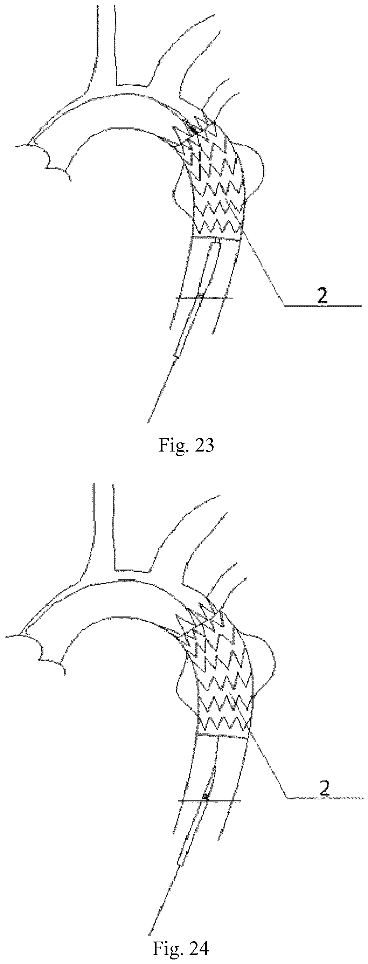

In the following description, reference will be made to Figs. 1 to 24, in which: Fig. 1 is a structural schematic of a stent delivery system in accordance with an embodiment of the present invention; Fig. 2 is an enlarged schematic view of the interior of an outer sheath in the stent delivery system in accordance with an embodiment of the present invention; Fig. 3 is a structural schematic of an outer sheath in the stent delivery system in accordance with an embodiment of the present invention; Fig. 4 is a structural schematic of a multi-stage deployment assembly in the stent delivery system in accordance with an embodiment of the present invention; Fig. 5 is a structural schematic of a multi-stage deployment base in the stent delivery system in accordance with an embodiment of the present invention; Fig. 6 is a structural schematic of a graft sleeve assembly in the stent delivery system in accordance with an embodiment of the present invention; Fig. 7 is a structural schematic of a rotary retraction mechanism in the stent delivery system in accordance with an embodiment of the present invention; Fig. 8 is a disassembled view of an anti-reverse rotation fastener and an outer sheath in the stent delivery system, which are to be assembled in a first manner, in accordance with an embodiment of the present invention; Fig. 9 shows the anti-reverse rotation fastener and the outer sheath in the stent delivery system, which are assembled together in the first manner, in accordance with an embodiment of the present invention; Fig. 10 is a disassembled view of an anti-reverse rotation fastener and an outer sheath the stent delivery system, which are to be assembled in a second manner, in accordance with an embodiment of the present invention; Fig. 11 shows the anti-reverse rotation fastener and the outer sheath in the stent delivery system, which are assembled together in the second manner, in accordance with an embodiment of the present invention; Fig. 12 is a structural schematic of a deployment structure in the stent delivery system in accordance with an embodiment of the present invention; Fig. 13 is an assembled plan view of a control disk in the stent delivery system in accordance with an embodiment of the present invention; Fig. 14 is a cross-sectional view of a deployment structure in the stent delivery system in accordance with an embodiment of the present invention; Fig. 15 is a structural schematic of a left handle knob half in the stent delivery system in accordance with an embodiment of the present invention; Fig. 16 is an assembled plan view of a handle knob in the stent delivery system in accordance with an embodiment of the present invention; Fig. 17 is a structural schematic of a control disk in the stent delivery system in accordance with an embodiment of the present invention; Fig. 18 is a structural schematic of a handle knob in the stent delivery system in accordance with an embodiment of the present invention; Fig. 19 schematically shows a first step of a method for implanting a stent using the stent delivery system in accordance with an embodiment of the present invention; Fig. 20 schematically shows a second step of the method for implanting a stent using the stent delivery system in accordance with an embodiment of the present invention; Fig. 21 schematically shows a third step of the method for implanting a stent using the stent delivery system in accordance with an embodiment of the present invention; Fig. 22 schematically shows a fourth step of the method for implanting a stent using the stent delivery system in accordance with an embodiment of the present invention; Fig. 23 schematically shows a fifth step of the method for implanting a stent using the stent delivery system in accordance with an embodiment of the present invention; and Fig. 24 schematically shows a sixth step of the method for implanting a stent using the stent delivery system in accordance with an embodiment of the present invention. In order to facilitate the explanation, as used herein, a "proximal end" refers to the end leading in the direction of advancement of the stent delivery system, i.e., the end farther from the operator, and a "distal end" refers to the end closer to the operator.

With emphasized reference to Figs. 1 and 2, as shown in Figs. 1 and 2, a stent delivery system according to an embodiment of the present invention is configured to be guided over a delivery guidewire to a target lesion site in an aorta and deploy a stent 2 at the site. The stent delivery system includes an outer sheath 100, a multi-stage deployment assembly 200, a graft sleeve assembly 300 and a handle assembly 400. The graft sleeve assembly 300 includes a graft sleeve 301, the stent 2 is crimped and received in the graft sleeve 301. The stent 2 has a bare segment 21 at its proximal end, which is constrained by the multi-stage deployment assembly 200. The graft sleeve 301 is disposed within the outer sheath 100, and the handle assembly 400 is connected to each of the outer sheath 100, the graft sleeve assembly 300 and the multi-stage deployment assembly 200. The handle assembly 400 is configured to control the outer sheath 100 and the graft sleeve assembly 300 so that the outer sheath 100 and the graft sleeve 301 of the graft sleeve assembly 300 move distally along their own respective axes to cause initial deployment of the stent 2. The handle assembly 400 is also configured to control the multi-stage deployment assembly 200 to deploy the proximal bare segment 21 of the stent 2 and thereby achieves final deployment of the stent 2.

Further, as shown in Fig. 3, the outer sheath 100 has a wall including, from inside outward, an inner layer 101, a reinforcing layer 102 and an outer layer 103. The reinforcing layer 102 includes at least one first reinforcing member 1021 and at least one second reinforcing member 1022. The first reinforcing member 1021 is helically wound about the axial direction of the outer sheath 100 and therefore also called a helical reinforcing member in this embodiment. The second reinforcing member 1022 extends along the axial direction of the outer sheath 100 and is therefore also referred to as an axial reinforcing member in this embodiment. Each of the inner layer 101 and the outer layer 103 made be formed of a single polymer material layer or multiple polymer material layers which are either homogeneous or heterogeneous layers.

Further, the helical reinforcing member may also be fabricated from a polymer material, for example, a sheet of liquid crystal polymer fibers in this embodiment which can minimize the fabrication cost of the outer sheath 100. Furthermore, the fibers in the helical reinforcing member may be selected as polyarylate fibers commercially available under the name Vectran, Unitika or Dnryl, or aramid fibers commercially available under the name Kevlar. In order to minimize the size of the outer sheath 100 to obtain an outer sheath 100 with a thinner wall, the helical reinforcing member is formed of a plurality of liquid crystal polymer fibers arranged in parallel into a sheet-like structure. Alternatively, the helical reinforcing member may also be formed of a metallic material, for example, one or more of nickel-titanium (NiTi), cobalt-chromium (CoCr) and platinum-iridium (Ptlr) alloys, gold, stainless steel, and tantalum.

Depending on the desired properties of the outer sheath 100, the fibers may be either fused into one or more strands or not. In the case where the fibers are fused into one or more strands, the outer sheath can provide mechanical properties similar to those of solid bars, while in the case where the fibers are not fused, it will provide mechanical properties similar to those of filaments. Differing from solid bars, filaments exhibit higher flexibility and higher fatigue resistance after repeated flexure and are therefore more conformable to the tortuous geometry of blood vessels.

The fibers may each have a rounded cross section with a diameter ranging from 0.0127mm to 0.254mm (from 0.0005 inches to 0.01 inches) . Further, the number of fibers in the helical reinforcing member may range from 2 to 40, with 20 being preferred, thereby ensuring a small size and sufficient radial strength of the helical reinforcing member.

Further, in order to minimize frictional resistance between the fibers in the helical reinforcing member and other components arising from, for example, burring or rupturing of the fibers during the fabrication of the outer sheath 100 due to the inevitable friction with manufacturing fixtures, or from rupturing of the fibers as a result of the friction of the outer sheath 100 with other components under the action of forces, the fibers may be surface-coated with a lubricating oil such as T-97, T-150 or the like.

In case of the helical reinforcing member being fabricated from a metallic material, inexpensive stainless steel that can provide desirable strength may be suitably selected. In order for a minimized size of the outer sheath 100, thin wall and high radial strength of the outer sheath 100 to be achieved, the helical reinforcing member may be woven from flat stainless steel wires each having a 0.0254mm ( 0.001 inches) to 0.127mm (0.005 inches) long, 0.0254mm to 0.127mm (0.001 inches to 0.005 inches) wide, rectangular cross section.

Further, the flat stainless steel wires may be woven with a thread count of 16, 32 or 64. Preferably, they are woven with a thread count of 16 in an over 2 under 2 pattern (i.e., 2/2 twill) to form the helical reinforcing member. Further, subsequent to the weaving of the stainless steel wires, a thin-walled heat-shrink tubing, such as a PET heat-shrink tubing with a wall thickness of 0.00635mm (0.00025 inches), may be provided to secure the free ends of the wires to prevent their warpage which may lead to bumps on interior and exterior surfaces of the outer sheath 100 at the part thereof formed by the ends.

Further, the helical reinforcing member may include at least one helical element. The helical reinforcing member may be formed of two or more helical elements along part or the whole of its length and of a single helical element along the remainder of the length. For example, the helical reinforcing member may axially have at least two sections, of which, at least one is formed of one helical element, and additional at least one is formed of a plurality of helical elements spiral to form a spiral woven mesh structure. The single helical element may be wounded into a spiral structure preferably with an axial winding density of 30-50 turns/2.54cm(inch) The two or more helical elements may be woven to form a mesh structure with an axial density generally of 40-80 PPI (intersection points per 2.54cm (inch)), with 60 PPI being preferred.

Such a design provides the following advantages: 1) the single helical element forming part of the outer sheath 100 imparts to the part sufficient bending flexibility which facilitates the delivery system to reach the target lesion site; 2) the two or more helical elements forming the remainder of the outer sheath 100 allows the part to maintain or have improved radial strength; and 3) the axial reinforcing member ensures sufficiently high axial strength and high radial strength for the outer sheath 100. For example, portions of the outer sheath 100 at its both ends (proximal and distal ends) may each consist of a plurality of helical elements, while the remainder of the outer sheath 100 may consist of only one helical element. Alternatively, portions each consisting of a plurality of helical elements may alternate with portions each consisting of a single helical element.

Further, the helical reinforcing member may consist of, along its entire length, two or more helical elements which form a woven mesh structure, and the axial reinforcing member is interwoven in the woven mesh structure along the axial direction of the outer sheath 100. In this embodiment, the helical reinforcing member consists of, along its entire length, two helical elements in contact respective with the inner and outer layers. This design with the axial reinforcing member disposed between the two or more helical elements will not cause bumps on the surface of the inner layer 101 or outer layer 103 of the outer sheath 100, which may introduce additional friction. Additionally, as the axial reinforcing member is not fixedly attached to the polymeric inner or outer layer, the increased frictional force caused by the bumps on the surface of the inner layer 101 and the outer layer 103 is avoided while maintaining the advantages of the axial reinforcing member, and at the same time the limited bendability caused by the fixing of the axial reinforcing member and the inner layer 101 or the outer layer 103 is also overcome.

Further, the axial reinforcing member may also be fabricated from a polymer material in the form of filaments, wires, fibers, monofilaments, multifilaments, cords, etc., with flat liquid crystal polymer fibers being preferred for a reduced cost of the outer sheath 100. In order to achieve both a minimized size and a thin wall of the outer sheath 100, the axial reinforcing member may be a flat structure formed of a plurality of fibers arranged in parallel. Each of the fibers may have a circular cross section with a diameter ranging from 0.0127mm to 0.254mm (from 0.0005 inches to 0.01 inches). Alternatively, each of the fibers may also have a rectangular cross section.

Alternatively, the axial reinforcing member may be fabricated from a metallic material, for example, formed of a metallic material, for example, one or more of NiTi, CoCr and Ptlr alloys, gold, stainless steel, and tantalum. In order to achieve a minimized size, a thin wall and a high radial strength of the outer sheath 100, the axial reinforcing member may be formed of flat stainless steel wires each having a 0.0254mm to 0.127mm ( 0.001 inches to 0.005 inches) long, 0.0254mm to 0.127mm ( 0.001 inches to 0.005 inches) wide, rectangular cross section.

Further, in order to minimize frictional resistance between the fibers in the axial reinforcing member and other components arising from, for example, burring or rupturing of the fibers during the fabrication of the outer sheath 100 due to the inevitable friction with manufacturing fixtures, or from rupturing of the fibers as a result of the friction of the outer sheath 100 with other components under the action of forces, the fibers may be surface-coated with a lubricating oil such as T-97, T-150 or the like.

A plurality of such axial reinforcing members may be provided which are disposed at equal angular intervals along a circumferential direction of the outer sheath. Specifically, the number of the axial reinforcing members may be 4, 8, 10, 12 or 16. In this case, adjacent axial reinforcing members are spaced apart from each other by an angle of 90°, 45°, 36°, 30° or 22.5° in the circumferential direction of the outer sheath 100. Such a design of the axial reinforcing members provides the following advantages: 1) the axial reinforcing members prevent axial extension of the outer sheath 100 so that when the physician pulls the proximal end of the outer sheath 100 during the surgical procedure, the distance the outer sheath moves axially is proportional to the pulling force applied by the physician; 2) the even arrangement of the axial reinforcing members impart to the outer sheath 100 uniform bending flexibility across its circumference, which is favorable to accurate control of the outer sheath 100 when the outer sheath 100 is being advanced within a tortuous blood vessel; 3) compared to the design with a single axial reinforcing member, the axial reinforcing members arranged equidistantly in the circumferential direction of the outer sheath 100 result in improved circumferential isotropy which reduces the possibility of bending of the outer sheath 100 under pressure from the blood flow or when loaded therein with an implant with extremely high expandability; 4) higher, uniform stiffness of the outer sheath 100 is achievable, allowing a lower cost of the outer sheath 100 by reducing the wall thickness of the outer sheath 100 or using softer outer layer in the outer sheath 100; and 5) the axial reinforcing members limit radial expansion of the outer sheath 100, thereby significantly increasing the breaking strength of the outer sheath 100.

In embodiments of the present invention, the inner layer 101 and the outer layer 103 may be made of either the same or different polymer materials. In particular, the inner layer 101 may include at least one polymer material layer (i.e., the inner layer 101 may be either a single-layer structure or a multilayer structure). Similarly, the outer layer 103 may also include at least one polymer material layer (i.e., the outer layer 103 may also be either a single-layer structure or a multilayer structure). The polymer material layer(s) may be formed of one or more of a fluoroplastic, a polyolefin, a polyether block amide resin, a polyamide or a polyurethane. Specific examples may include fluoroplastics such as PTFE, FEP, PFA, PCTFF, ECTFE, ETFE, PVDF, PVF and EFEP and polyolefins such as LDPE, HDPE and UHMWPE. Preferably, the inner layer 101 may have a wall thickness ranging from 0.0127mm to 0.1016mm (0.0005 inches to 0.004 inches), and the outer layer 103 may have a wall thickness ranging from 0.0254mm to 0.0762mm ( 0.001 inches to 0.003 inches).

Preferably, the inner layer 101 may be provided with a hydrophilic or hydrophobic coating on its interior surface. The exterior surface of the inner layer 101 may be subjected to a surface finish treatment for increasing the activity of the exterior surface of the inner layer 101. For example, the exterior surface may be treated with plasma or an etchant.

Preferably, the exterior surface of the outer layer 103 may be coated with a hydrophilic or hydrophobic substance or a self-lubricating fluoroplastic. The hydrophilic coating may be selected as PVP, the hydrophobic coating as a silicone oil and the self-lubricating fluoroplastic as PTFE or the like which can reduce the relatively great frictional resistance between the outer sheath 100 and the blood vessel wall. Further, in order for sufficient attachment/adhesion to be obtained between the outer layer 103 and the coating, the exterior surface of the outer layer 103 may be in advance subjected to a surface finish treatment using, for example, plasma or an etchant, for increasing the activity of the exterior surface of the outer layer 103. Further, as shown in Fig. 4, the multi-stage deployment assembly 200 includes a conical tip 201, a multi-stage deployment anchor 202, a multi-stage deployment base 203, several multi-stage deployment rods 204 and multi-stage deployment guidewires 205. A proximal end of the conical tip 201, i.e., the end with a reduced diameter, serves to guide the advancement of the delivery system. A distal end of the conical tip 201, i.e., the end with a larger diameter, defines several securing bores 2011 for receiving the respective multi-stage deployment rods 204. The multi-stage deployment anchor 202 defines several guide bores 2021 allowing passage therethrough of the respective multi-stage deployment rods 204. Each of the several multi-stage deployment rods 204 has a distal end in fixed connection to the multi-stage deployment base 203 and a proximal end that passes through a corresponding one of the guide bores 2021 of the multi-stage deployment anchor 202 and is received within a corresponding one of the securing bores 2011 of the conical tip 201. The multi-stage deployment guidewires 205 each have a proximal end in fixed connection with the multi-stage deployment base 203. The multi-stage deployment guidewires 205 each have a distal end coupled to the handle assembly 400. Segments of the multi-stage deployment rods 204 between the conical tip 201 and the multi-stage deployment anchor 202 (Space A) are configured to be inserted through and thereby constrain the proximal bare segment 21 of the stent 2. In this embodiment, an axial length of Space A is made as small as possible while allowing satisfactory assembly and deployment, in order to enable the segments of the multi-stage deployment rods 204 therein to have increased stiffness and experience less deformation resulting from the expansion of the stent 2.

In order to load the stent-graft 2, the segments of the multi-stage deployment rods 204 within Space A is inserted through mesh holes in the proximal bare segment 21 of the stent 2, and the multi-stage deployment base 203 is pushed toward the conical tip 201 until it reaches its limit of movement. The multi-stage deployment rods 204 are then inserted into the securing bores 2011 at the diametrically larger end of the conical tip 201 under the guidance of the guide bores 2021 of the multi-stage deployment anchor 202, so that the multi-stage deployment rods 204 are retained by the conical tip 201 and the multi-stage deployment anchor 202, and the proximal bare segment 21 of the stent 2 is secured. In order to deploy the stent 2, the multi-stage deployment guidewires 205 are pulled back via the handle assembly 400, dislodging the multi-stage deployment rods 204 from the securing bores 2011 of the conical tip 201 and hence deploying the proximal bare segment 21 of the stent 2. Following this, the bare segment 21 of the stent 2 expands due to its own resilience and firmly presses against the blood vessel wall so that the stent 2 is anchored.

Further, each of the conical tip 201, the multi-stage deployment anchor 202 and the multi-stage deployment base 203 defines a channel through which an inner tube 1 is inserted. The inner tube 1 has a proximal end in connection with the conical tip 201. A limiting block 206 is provided over a portion of the inner tube 1 on a distal side of the multi-stage deployment base 203. The limiting block 206 has a greater diameter than the channel 2031 in the multi-stage deployment base 203, thereby limiting a displacement of the multi-stage deployment base 203 during deployment of the bare segment 21 of the stent 2, because excessive advancement of the multi-stage deployment base may cause it to come into contact and hence damage the nearby blood vessels.

Further, as shown in Fig. 5, the channel 2031 is formed at a center of the multi-stage deployment base 203 and surrounded by several uniformly distributed first bores 2032 for anchoring the corresponding multi-stage deployment rods 204. In this embodiment, the number of the first bores 2032 is six and the six first bores 2032 are distributed along a circumferential direction of the channel 2031 in an equiangular manner. Moreover, the number of the multi-stage deployment rods is accordingly six and the six multi-stage deployment rods 204 are anchored in the respective six first bores 2032. Around the channel 2031, there is provided at least one second bore 2033 for securing the multi-stage deployment guidewires 205. In this embodiment, the number of the second bore(s) 2033 is two and the number of the multi-stage deployment guidewires 205 is also two. Each of the multi-stage deployment guidewires is inserted through one of the second bores 2033 from the distal side of the multi-stage deployment base 203 and folded back on a proximal side of the multi-stage deployment base 203 and passes through the other second bore 2033. The multi-stage deployment guidewires 205 are fixed in the second bores 2033. The fixations of the multi-stage deployment guidewires 205 are achieved by, for example, but not limited to, welding, gluing or mechanical locking.

Further, each adjacent two of the six first bores 2032 may be paired and interconnected with an arcuate bore within the multi-stage deployment base so that three U-shaped bores are formed. Accordingly, each adjacent two of the six multi-stage deployment rods 204 may be paired and interconnected with an arcuate multi-stage deployment rod disposed within a respective one of the U-shaped bores of the multi-stage deployment base 203, so that three U-shaped multi-stage deployment rods are formed. The U-shaped multi-stage deployment rods 204 pass through the U-shaped bores, which can enhance the connection between the multi-stage deployment rods 204 and the multi-stage deployment base 203.

Further, as shown in Fig. 6, the graft sleeve assembly 300 includes the graft sleeve 301, a graft sleeve connecting tube 302 and a graft deployment guidewire 303. The graft sleeve connecting tube 302 has a proximal end in fixed connection with a distal end of the graft sleeve 301. The graft deployment guidewire 303 has a proximal end in fixed connection with a distal end of the graft sleeve connecting tube 302. A distal end of the graft deployment guidewire 303 is coupled to the handle assembly 400. The graft sleeve 301 is a soft sheathing sleeve made of a graft material and is configured to facilitate bending of the stent-graft 2 during its deployment. The graft sleeve connecting tube 302 is configured to guide the deployment of the graft sleeve 301. During the deployment of the graft sleeve 301, the graft deployment guidewire 303 is pulled via the handle assembly 400, causing the graft sleeve connecting tube 302 to guide an axial distal travel of the graft sleeve 301 which results in initial deployment of the stent 2 within the graft sleeve 301.

Further, the handle assembly 400 includes a rotary retraction mechanism and a deployment structure. The rotary retraction mechanism is in rotatable connection with the outer sheath 100 via a slider 403. The rotary retraction mechanism rotates to cause axial retraction of the outer sheath 100, thereby deploying the graft sleeve 301. Under the control of the deployment structure exerted via the graft deployment guidewire 303, the graft sleeve 301 retracts axially to initially deploy the stent 2 and the multi-stage deployment assembly 200 deploys the proximal bare segment 21 of the stent 2 to complete the deployment of the stent 2.

Further, as shown in Fig. 7, the rotary retraction mechanism may include a rear handle portion 401, an outer sheath connector 402, the slider 403 and guide rods 404. The slider 403 defines guide bores extending parallel to the axial direction of the outer sheath 100, and the guide rods 404 are disposed within the rear handle portion 401 so that they are oriented along the axial direction of the outer sheath 100 and inserted through the guide bores. The outer sheath 100 is axially connected to the slider 403 via the outer sheath connector 402, and the slider 403 is in threaded connection with the rear handle portion 401. When the rear handle portion 401 is rotated, the slider 403 will cause the outer sheath 100 to move axially along the guide rods 404, causing deployment of the graft sleeve 301 within the outer sheath 100 and concurrent slight expansion of the stent 2 within the graft sleeve 301.

Further, an exterior surface of the slider 403 may be provided with an external thread which engages an internal thread formed on an interior surface of the rear handle portion 401. When the rear handle portion 401 is rotated, the slider 403 will slide along the guide rod 404 and cause an axial travel of the outer sheath 100.

Further, the number of the guide rods 404 is two and the two guide rods 404 are symmetric to each other with respect to a center line of the slider 403. With the two guide rods 404, the slider 403 can make more reliable axial movement, which can enhance the deployment accuracy of the graft sleeve 301.

Further, the rotary retraction mechanism may further include an anti-reverse rotation fastener 405. The anti-reverse rotation fastener 405 is disposed over the outer sheath connector 402. The anti-reverse rotation fastener 405 has anti-reverse rotation elastic blades 4051 and one-way tooth slots 4041 are provided in portions of the guide rods 404 proximal to the anti-reverse rotation elastic blades 4051. Tips of the anti-reverse rotation elastic blades 4051 proximal to the one-way tooth slots 4041 are complementary in shape to notches between the one-way tooth slots 4041. During distal movement of the anti-reverse rotation fastener 405, the anti-reverse rotation elastic blades 4051 repeatedly deform and slide over bevels of the one-way tooth slots 4041, allowing a smooth travel of the outer sheath 100. Any possible proximal movement of the anti-reverse rotation fastener 405 is prevented because the one-way tooth slots 4041 have a limiting effect on the anti-reverse rotation elastic blades 4051 in this direction. As a result, proximal movement of the outer sheath 100 is blocked, avoiding medical accidents due to possible mistakes of the operator.

In this embodiment, the number of the anti-reverse rotation elastic blades 4051 is two and they come in contact with the respective guide rods 404 to constitute the anti-reverse rotation design. With this design, the anti-reverse rotation fastener 405 gains increased reliability.

In one embodiment of the present invention, as shown in Figs. 8 and 9, the anti-reverse rotation fastener 405 includes a connecting feature 4052. The anti-reverse rotation elastic blades 4051 are disposed on the connecting feature 4052. The connecting feature 4052 is an annular structure defining a notch 4053 extending parallel to the axial direction of the outer sheath 100. Accordingly, an I-shaped protrusion 4021 projects from the surface of the outer sheath connector 402 and has a vertical piece oriented parallel to the axial direction of the outer sheath 100. The notch 4053 can be slightly enlarged through applying opposite forces to the opposing edges thereof so that the annular structure can be fitted over the outer circumference of the outer sheath connector 402 in slight interference fit, with the opposing edges of the notch 4053 abutting against opposite faces of the vertical piece of the I-shaped protrusion 4021. With such a design, rotation of the annular structure is limited by the vertical piece of the I-shaped protrusion 4021, and its axial translation is limited by upper and lower horizontal pieces of the I-shaped protrusion 4021.

In an alternative embodiment of the present invention, as shown in Figs. 10 and 11, the anti-reverse rotation fastener 405 includes a connecting feature 4052. The anti-reverse rotation elastic blades 4051 are disposed on the connecting feature 4052. The connecting feature 4052 is an annular structure defining an L-shaped notch 4054 having a longitudinal portion extending parallel to the axial direction of the outer sheath 100 and a transverse portion extending perpendicular to the axial direction of the outer sheath 100. Accordingly, a protrusion 4022 projects from the surface of the outer sheath connector 402. The annular structure may be fitted over the outer circumference of the outer sheath connector 402, with a slight interference fit established therebetween, by axially advancing the protrusion 4022 within the longitudinal portion of the L-shaped notch 4054 until it reaches the opposing end wall thereof and then rotating the annular structure so that the protrusion 4022 comes in contact with the opposing end wall of the transverse portion of the L-shaped notch 4054. In this design, movement of the annular structure can be limited in the axial direction and in one circumferential direction. However, as the anti-reverse rotation fastener 405 will not be subject to any constraints in the other circumferential direction during use, the protrusion 4022 of the outer sheath connector 402 may be glued to the connecting feature 4052 to limit its movement in this direction.

The rotary retraction mechanism may further include a front handle portion 406. The front handle portion 406 is coupled to the rear handle portion 401 along the axial direction of the outer sheath 100, with a locking feature 4061 enabling/preventing relative movement between the slider 403 and the rear handle portion 406 to avoid mistakes of the operator. In this embodiment, the locking feature 4061 is a switch disposed on the end of the front handle portion 406 proximal to the rear handle portion 401. The switch can be pushed toward the rear handle portion 401 and partially inserted into a corresponding locking slot in the rear handle portion 401, thereby limiting the rotation of the rear handle portion 401 relative to the front handle portion 406. In this way, relative movement between the slider 403 and the rear handle portion 406 can be enabled (ON)/prevented (OFF).

Further, as shown in Figs. 12 and 13, the deployment structure includes a handle knob 407, a control disk 408, an end cover 409, a first lug 410 and a second lug 411. As illustrated, the handle knob 407 is disposed at a distal end of the rear handle portion 401. The control disk 408 is an annular structure fitted over and in rotatable connection with the handle knob 407. The end cover 409 is disposed on the handle knob 407 and configured to limit the movement of the control disk 408 along the axial direction of the outer sheath 100. Two semicircular grooves 4071, which extend along the axial direction of the outer sheath 100, are defined in a portion of the handle knob 407 proximal to the control disk 408. The first and second lugs 410, 411 have respective lug shafts 4101, 4111 which pass through the end cover 409 and are received in the two semicircular grooves 4071. The lug shafts 4101, 4111 of the first and second lugs 410, 411 define respective limiting protrusions 4102, 4112 both projecting away from a center of the control disk 408. The dislodgement of the lug shafts 4101, 4111 is blocked because an inner diameter of the control disk 408 is designed to limit translation of the limiting protrusions 4102, 4112 along the axial direction of the outer sheath 100 so as to avoid dislodgement of the lug shafts 4101, 4111 from the semicircular grooves 4071. In addition, a deployment recess 4081, formed in an inner surface of the control disk 408, is complementary in shape to the limiting protrusions 4102, 4112. When the deployment recess 4081 is rotated into positional coincidence with one of the semicircular grooves 4071, the one of the lug shafts 4101, 4111 received in this semicircular groove 4071 can be pulled off therefrom. The lug shafts 4101, 4111 of the first and second lugs 410, 411 are connected to the graft sleeve assembly 300 and the multi-stage deployment assembly 200, respectively, via the graft deployment guidewire 303 and the multi-stage deployment guidewire 205, respectively, so that they can effectuate independent deployment control.

Further, an arrow may be marked on the surface of the handle knob 407, and the numerals "1" and "2" on the surface of the control disk 408. When the control disk 408 is turned to cause alignment of the numeral "1" with the arrow, the deployment recess 4081 will coincide in position with the one of the semicircular grooves 4071 in which the lug shaft 4101 of the first lug 410 is received and the lug shaft 4101 of the first lug 410 can be pulled off from the semicircular groove 4071 to retract the graft deployment guidewire 303, thereby enabling initial deployment of the stent 2. Similarly, when the numeral "2" is aligned with the arrow, the deployment recess 4081 will coincide in position with the one of the semicircular grooves 4071 in which the lug shaft 4111 of the second lug 411 is received and the lug shaft 4111 of the second lug 411 can be pulled off from the semicircular groove 4071, resulting in retraction of the multi-stage deployment guidewire 205 and hence accomplishment of final deployment of the stent 2. Such a design can effectively avoid errors that may arising during the deployment and enhance deployment accuracy of the stent 2.

Further, the first and second lugs 410, 411 may further include respective lug handles 4103, 4113 and respective limiting members 4104, 4114. The lug handles 4103, 4113 are connected to the respective lug shafts 4101, 4111 which are inserted through the respective limiting members 4104, 4114. The limiting member 4114 of the second lug 411 is located closer than the limiting member 4104 of the first lug 410 to the end cover 409. Projections of the limiting members 4104, 4114 of the first and second lugs 410, 411 along the axial direction of the outer sheath 100 partially overlap each other. With such a design of the limiting members 4104, 4114, the lug shaft 4111 of the second lug 411 cannot be pulled off from the semicircular groove 4071 before the lug shaft 4101 of the first lug 410 is pulled off. This adds security to the deployment process.

Further, as shown in Figs. 14 to 16, the handle knob 407 may include a left handle knob half 4072 and a right handle knob half 4073. Two left semicircular grooves extending along the axial direction of the outer sheath 100 are defined in the left handle knob half 4072, and two right semicircular grooves extending along the axial direction of the outer sheath 100 are defined in the right handle knob half 4073. In addition, the left semicircular grooves are provided with radially projecting assembly pins 4075, and the right semicircular grooves are provided with radially extending assembly holes. The left handle knob half 4072 can be assembled with the right handle knob half 4073 by inserting the assembly pins 4075 into the assembly holes. When assembled, the two left semicircular grooves and the two right semicircular grooves form two bores 4074 for respectively guiding the graft deployment guidewire 303 and the multi-stage deployment guidewire 205. Portions of the assembly pins 4075 remaining in the bores 4074 can prevent the graft deployment guidewire 303 or the multi-stage deployment guidewire 205 from being stuck in a gap 4076 between the left handle knob half 4072 and the right handle knob half 4073, which may lead to loss of maneuverability of the graft deployment guidewire 303 or the multi-stage deployment guidewire 205 and hence failure in the deployment of the stent 2.

Further, as shown in Figs. 17 and 18, one-way teeth 4082 may be provided along the inner circumference of the control disk 408, while a one-way elastic blade 4077 may project from the outer circumference of the handle knob 407. The one-way elastic blade 4077 may have a tip, proximate to the one-way teeth 4082, complementary in shape to notches between the one-way teeth 4082. With such a design, the control disk 408 can be turned in a certain direction with the one-way elastic blade 4077 repeatedly deforming and sliding over the one-way teeth 4082, but its rotation in the opposite direction will be blocked because the one-way elastic blade 4077 will be pressed against one of the one-way teeth 4082 and could not move any longer. This design can add resistance to the operation of the control disk 408, which can improve operation accuracy and prevent mistakes of the operator. In this embodiment, the control disk 408 can be rotated only clockwise to facilitate the operator's operations.

Further, each of the handle knob 407, the control disk 408 and the end cover 409 may define channels for the delivery guidewire to pass therethrough.

With emphasized reference to Figs. 19 to 24, the present disclosure also provides a method, that is not part of the invention, for using the stent delivery system as defined above, including the steps of:
1) as shown in Fig. 19, introducing the stent 2, the graft sleeve 301, the outer sheath 100 and the multi-stage deployment assembly 200 over the delivery guidewire 1 into a target lumen;
2) as shown in Fig. 20, manipulating the handle assembly 400 to advance the outer sheath 100 axially distally, thereby deploying the graft sleeve 301 from the graft sleeve assembly 300, which results in slight expansion of the stent 2;
3) as shown in Fig. 21, in conjunction and comparison with Fig. 20, further advancing the stent 2, the graft sleeve 301 and the multi-stage deployment assembly 200 over the delivery guidewire to a target site in the target lumen;
4) as shown in Fig. 22, manipulating the handle assembly 400 to control the graft sleeve assembly 300 so that the graft sleeve 301 therein advances axially distally, resulting in initial deployment of the stent 2 in the graft sleeve 301 with the proximal end of the stent 2 still being retained by the multi-stage deployment assembly 200;
5) as shown in Fig. 23, manipulating the handle assembly 400 to control the multi-stage deployment assembly 200 so that the proximal bare segment 21 of the stent 2 is deployed, accomplishing final deployment of the stent 2; and
6) as shown in Fig. 24, retracting the graft sleeve 301, the outer sheath 100 and the multi-stage deployment assembly 200 over the delivery guidewire.

Specifically, in step 2), the deployment of the graft sleeve 301 may be accomplished by switching the switch on the front handle portion 406 (i.e., locking feature 4061) to the "ON" position and rotating the rear handle portion 401 to retract the outer sheath 100.

In step 4), the initial deployment of the stent 2 may be accomplished by rotating the control disk 408 to cause alignment of the numeral "1" with the arrow mark on the handle knob 407 and pulling the first lug 410 to cause the graft deployment guidewire 303 to retract the graft sleeve 301.

In step 5), the final deployment of the stent 2 may be accomplished by applying fine positional adjustments to the stent 2 if needed, rotating the control disk to align the numeral "2" with the arrow mark on the handle knob 407 and pulling the second lug 411 and hence the multi-stage deployment guidewire 205 to cause the multi-stage deployment rods 204 to dislodge from the securing bores 2011. As a result, the proximal bare segment 21 of the stent 2 disengages from the rod segments in Space A and expands.

In step 6), the inner tube 1 may be first retracted until the conical tip 201 is received in the outer sheath 100, and the outer sheath 100 may then be retracted along with all the other components inserted therethrough so that the stent delivery system is entirely withdrawn from the body.

In summary, the stent delivery system according to the embodiments of the present invention is capable of more reliable deployment by virtue of the design of the outer sheath 100, the multi-stage deployment assembly 200, the graft sleeve assembly 300 and the handle assembly 400 and the design of the connections between these components. The method proposed, which does not form part of the invention, for using the delivery system involves, sequentially, deployment of the graft sleeve 301, initial deployment of the stent 2 and final deployment of the stent 2, accomplished respectively by the outer sheath 100, the graft sleeve assembly 300 and the multi-stage deployment assembly 200 under the control of the handle assembly 400. This method, in combination with the overall design of the delivery system, allows increased stent deployment accuracy of the delivery system and reduced possibility of medical accidents.

It is apparent that those skilled in the art can make various modifications and variations to the present invention without departing from scope of the invention, which is defined by the claims.

## Claims

1. A handle assembly (400) of a stent delivery system, comprising a rear handle portion (401), a slider (403) and two guide rods (404), wherein the rear handle portion (401) is in threaded connection with the slider (403), the slider (403) in slidable connection with the two guide rods (404), the two guide rods (404) disposed in parallel to an outer sheath (100) for accommodating a stent, and the two guide rods (404) are symmetric to each other with respect to an axis of the slider (403), the slider (403) in axial connection with the outer sheath (100), and the slider (403) is provided with an external thread on an exterior surface thereof, which engages an internal thread on an interior surface of the rear handle portion (401), and wherein when the rear handle portion (401) is rotated, the slider (403) travels along the two guide rods (404) and causes axial movement of the outer sheath (100),
wherein the outer sheath (100) comprises an inner layer (101), a reinforcing layer (102) and an outer layer (103) which are arranged from inside outward, wherein the reinforcing layer (102) comprises at least one first reinforcing member (1021) and at least one second reinforcing member (1022), the first reinforcing member (1021) spiraling along an axis of the outer sheath (100), the second reinforcing member (1022) extending parallel to the axis of the outer sheath (100).

2. The handle assembly (400) of claim 1, further comprising an outer sheath connector (402) and an anti-reverse rotation fastener (405), the outer sheath connector (402) being in connection with the outer sheath (100) and the slider (403) at respective ends thereof, the anti-reverse rotation fastener (405) being disposed on the outer sheath connector (402) and being unidirectionally movable along the two guide rods (404).

3. The handle assembly (400) of claim 2, wherein the anti-reverse rotation fastener (405) comprises anti-reverse rotation elastic blades (4051), wherein one-way tooth slots (4041) are provided in portions of the two guide rods (404) proximal to the anti-reverse rotation elastic blades (4051), wherein the anti-reverse rotation elastic blades (4051) has tips that are proximal to the one-way tooth slots (4041) and complementary in shape to notches between the one-way tooth slots (4041), wherein when the anti-reverse rotation fastener (405) moves distally, the anti-reverse rotation elastic blades (4051) repeatedly deforming and sliding over the one-way tooth slots (4041), allowing a smooth travel of the outer sheath (100), and wherein any possible proximal movement of the anti-reverse rotation fastener (405) is prevented due to a limiting effect of the one-way tooth slots (4041) on the anti-reverse rotation elastic blades (4051) to prevent the outer sheath (100) from moving proximally.

4. The handle assembly (400) of claim 3, wherein the anti-reverse rotation fastener (405) further comprises a connecting feature (4052), the anti-reverse rotation elastic blades (4051) disposed on the connecting feature (4052), and wherein the connecting feature (4052) is an annular structure fitted over the outer sheath connector (402).

5. The handle assembly (400) of claim 4, wherein the annular structure defines a notch (4053) extending parallel to an axial direction of the outer sheath (100), wherein an I-shaped protrusion (4021) projects from a surface of the outer sheath connector (402) and comprises a vertical piece oriented parallel to the axial direction of the outer sheath (100), and wherein the annular structure is fitted over and thereby fixed to the outer sheath connector (402) with opposing edges of the notch (4053) abutting against opposite faces of vertical piece of the I-shaped protrusion (4021).

6. The handle assembly (400) of claim 4, wherein the annular structure defines an L-shaped notch (4054) comprising a longitudinal portion extending parallel to an axial direction of the outer sheath (100) and a transverse portion extending perpendicular to the axial direction of the outer sheath (100), wherein a protrusion (4022) projects from a surface of the outer sheath connector (402), and wherein annular structure is fitted over the outer sheath connector (402) by advancing the protrusion (4022) within the longitudinal portion of the L-shaped notch (4054) along the axial direction of the outer sheath until the protrusion (4022) reaches the opposing end wall of the longitudinal portion and then rotating the annular structure so that the protrusion (4022) comes in contact with the opposing end wall of the transverse portion and is thereby limited in translation in the axial direction of the outer sheath (100) and rotation in one circumferential direction.

7. The handle assembly (400) of any one of claims 1 to 6, further comprising a front handle portion (406) coupled to the rear handle portion (401) along an axial direction of the outer sheath (100), with a locking feature (4061) enabling or preventing relative movement between the slider (403) and the rear handle portion (401).

8. The handle assembly (400) of claim 7, wherein the locking feature (4061) is a switch disposed on an end of the front handle portion (406) proximal to the rear handle portion (401), and wherein when the switch is pushed toward the rear handle portion (401), part of the switch is inserted into a corresponding locking slot in the rear handle portion (401), thereby limiting rotation of the rear handle portion (401) relative to the front handle portion (406) and thus preventing relative movement between the slider (403) and the rear handle portion (401).

9. The handle assembly (400) of claim 1, wherein a graft sleeve (301) is provided between the stent and the outer sheath (100), and wherein the handle assembly further comprises a first lug (410) in axial connection with the graft sleeve (301) via a graft deployment guidewire (303).

10. The handle assembly (400) of claim 9, wherein the stent has a proximal end constrained by a multi-stage deployment assembly (200), wherein the handle assembly (400) further comprises a second lug (411) in axial connection with the multi-stage deployment assembly (200) via multi-stage deployment guidewires (205), and wherein the multi-stage deployment assembly (200) comprises a conical tip (201), a multi-stage deployment anchor (202), a multi-stage deployment base (203), a plurality of multi-stage deployment rods (204) and multi-stage deployment guidewires (205), the conical tip (201) having a distal end defining a plurality of securing bores (2011) for receiving the corresponding multi-stage deployment rods (204), the multi-stage deployment anchor (202) defining a plurality of guide bores (2021) allowing passage therethrough of the corresponding multi-stage deployment rods (204), the plurality of multi-stage deployment rods (204) each having a distal end in fixed connection with the multi-stage deployment base (203) and a proximal end that passes through a corresponding one of the guide bores (2021) of the multi-stage deployment anchor (202) and received within a corresponding one of the securing bores (2011) of the conical tip (201), the multi-stage deployment guidewires (205) each having a proximal end in fixed connection with the multi-stage deployment base (203), the multi-stage deployment rods (204) having segments between the conical tip (201) and multi-stage deployment anchor (202), which are configured to be inserted through and thereby constrain a proximal end of a stent.

11. The handle assembly (400) of claim 10, further comprising a handle knob (407), a control disk (408) and an end cover (409), the handle knob (407) in connection with a distal end of the rear handle portion (401), the control disk (408) being an annular structure fitted over and in rotatable connection with the handle knob (407), the end cover (409) disposed at a distal end of the handle knob (407) and configured to limit a movement of the control disk (408) along an axial direction of the outer sheath (100).

12. The handle assembly (400) of claim 11, wherein two grooves (4071) having semicircular cross sections, which extend along the axial direction of the outer sheath (100), are defined in a portion of the handle knob (407) proximal to the control disk (408), wherein the first and second lugs (410, 411) comprise respective lug shafts (4101, 4111) which pass through holes in the end cover (409) and are received in the two grooves (4071), wherein the lug shafts (4101, 4111) of the first and second lugs (410, 411) define respective limiting protrusions (4102, 4112) both projecting away from a center of the control disk (408), wherein an inner diameter of the control disk (408) is designed to limit translation of the limiting protrusions (4102, 4112) along the axial direction of the outer sheath (100) so as to avoid dislodgement of the lug shafts (4101, 4111) from the two grooves (4071), wherein a deployment recess (4081) formed in an inner surface of the control disk (408) is complementary in shape to the limiting protrusions (4102, 4112), and wherein when the deployment recess (4081) is rotated into positional coincidence with one of the two grooves (4071), the one of the lug shafts (4101, 4111) received in the one of the two grooves (4071) is able to be pulled off therefrom.

13. The handle assembly (400) of claim 11 or 12, wherein one-way teeth (4082) are provided along an inner circumference of the control disk (408), wherein a one-way elastic blade (4077) with a tip that is complementary in shape to notches between the one-way teeth (4082) projects from an outer circumference of the handle knob (407), and wherein when the control disk (408) rotates in one direction, the one-way elastic blade (4077) repeatedly deforming and sliding over the one-way teeth (4082), allowing a smooth rotation of the control disk (408), and wherein any possible rotation of the control disk (408) in an opposite direction is prevented due to a limiting effect of the one-way teeth (4082) on the one-way elastic blade (4077) to prevent the control disk (408) from rotating.

14. The handle assembly (400) of claim 11 or 12, wherein the handle knob (407) comprises a left handle knob half (4072) and a right handle knob half (4073), the left handle knob half (4072) defining two left semicircular grooves extending along the axial direction of the outer sheath (100), the right handle knob half (4073), at corresponding positions thereof, defining two right semicircular grooves extending along the axial direction of the outer sheath (100), the left semicircular grooves provided with radially projecting assembly pins (4075), the right semicircular grooves, at corresponding positions thereof, provided with radially extending assembly holes, wherein when the assembly pins (4075) are inserted into the assembly holes, the left handle knob half (4072) is assembled with the right handle knob half (4073) and the two left semicircular grooves and the two right semicircular grooves form two bores (4074) through which the graft deployment guidewire (303) and the multi-stage deployment guidewires (205) are correspondingly inserted through, with portions of the assembly pins (4075) remaining in the bores (4074) preventing any of the guidewires from being stuck in a gap (4076) between the left handle knob half (4072) and the right handle knob half (4073).

15. The handle assembly (400) of claim 11 or 12, wherein the first and second lugs (410, 411) further comprises respective lug handles (4103, 4113) and respective limiting members (4104, 4114), the lug handles (4103, 4113) connected to the corresponding lug shafts (4101, 4111) which are inserted through the corresponding limiting members (4104, 4114), the limiting member (4104, 4114) of the first lug (410) configured to limit distal movement of the limiting member (4104, 4114) of the second lug (411).

16. The handle assembly (400) of claim 15, wherein there is a partial overlap between projections of the limiting members (4104, 4114) of the first and second lugs (410, 411) along the axial direction of the outer sheath (100), and wherein a portion of the limiting member (4104, 4114) of the second lug (411) that contributes to the overlap is disposed proximally with respect to a portion of the limiting member (4104, 4114) of the first lug (410) that contributes to the overlap.

17. A stent delivery system, comprising an outer sheath (100), a multi-stage deployment assembly (200), a graft sleeve assembly (300) and the handle assembly (400) as defined in any one of claims 1 to 16, the graft sleeve assembly (300) comprising a graft sleeve (301) in which a stent is crimped and received, the stent having a proximal end constrained by the multi-stage deployment assembly (200), the graft sleeve (301) disposed within the outer sheath (100), the handle assembly (400) in connection with each of the outer sheath (100), the graft sleeve assembly (300) and the multi-stage deployment assembly (200),
wherein the multi-stage deployment assembly (200) comprises a conical tip (201), a multi-stage deployment anchor (202), a multi-stage deployment base (203), a plurality of multi-stage deployment rods (204) and multi-stage deployment guidewires (205), the conical tip (201) having a distal end defining a plurality of securing bores (2011) for receiving the corresponding multi-stage deployment rods (204), the multi-stage deployment anchor (202) defining a plurality of guide bores (2021) allowing passage therethrough of the corresponding multi-stage deployment rods (204), the plurality of multi-stage deployment rods (204) each having a distal end in fixed connection with the multi-stage deployment base (203) and a proximal end that passes through a corresponding one of the guide bores (2021) of the multi-stage deployment anchor (202) and received within a corresponding one of the securing bores (2011) of the conical tip (201), the multi-stage deployment guidewires (205) each having a proximal end in fixed connection with the multi-stage deployment base (203), the multi-stage deployment rods (204) having segments between the conical tip (201) and multi-stage deployment anchor (202), which are configured to be inserted through and thereby constrain a proximal end of a stent.

## Patentansprüche

1. Griffanordnung (400) eines Stenteinführungssystems, umfassend ein hinteres Griffteil (401), einen Schieber (403) und zwei Führungsstangen (404), wobei das hintere Griffteil (401) mit dem Schieber (403) in Gewindeverbindung ist, wobei der Schieber (403) mit den zwei Führungsstangen (404) in verschiebbarer Verbindung ist, wobei die zwei Führungsstangen (404) parallel zu einer Außenhülle (100) zur Aufnahme eines Stents angeordnet sind, und wobei die zwei Führungsstangen (404) bezüglich einer Achse des Schiebers (403) symmetrisch zueinander sind, wobei der Schieber (403) mit der Außenhülle (100) in axialer Verbindung ist, und wobei der Schieber (403) auf einer Außenfläche desselben mit einem Außengewinde versehen ist, die in ein Innengewinde auf einer Innenfläche des hinteren Griffteils (401) eingreift, und wobei, wenn das hintere Griffteil (401) rotiert wird, der Schieber (403) sich entlang der zwei Führungsstangen (404) bewegt und eine axiale Bewegung der Außenhülle (100) bewirkt,
wobei die Außenhülle (100) eine Innenschicht (101), eine Verstärkungsschicht (102) und eine Außenschicht (103) umfasst, die von innen nach außen angeordnet sind, wobei die Verstärkungsschicht (102) mindestens ein erstes Verstärkungselement (1021) und mindestens ein zweites Verstärkungselement (1022) umfasst, wobei das erste Verstärkungselement (1021) spiralförmig entlang einer Achse der Außenhülle (100) verläuft, wobei sich das zweite Verstärkungselement (1022) parallel zur Achse der Außenhülle (100) erstreckt.

2. Griffanordnung (400) nach Anspruch 1, ferner umfassend einen Außenhüllenverbinder (402) und einen Anti-Rückrotationsbefestiger (405), wobei der Außenhüllenverbinder (402) in Verbindung mit der Außenhülle (100) und dem Schieber (403) an jeweiligen Enden desselben ist, wobei der Anti-Rückrotationsbefestiger (405) an dem Außenhüllenverbinder (402) angeordnet ist und entlang der zwei Führungsstangen (404) in unidirektionaler Richtung bewegbar ist.

3. Griffanordnung (400) nach Anspruch 2, wobei der Anti-Rückrotationsbefestiger (405) elastische Anti-Rückrotationsklingen (4051) umfasst, wobei Einwegzahnschlitze (4041) in Abschnitten der beiden Führungsstangen (404) proximal zu den elastischen Anti-Rückrotationsklingen (4051) vorgesehen sind, wobei die elastischen Anti-Rückrotationsklingen (4051) Spitzen aufweisen, die proximal zu den Einwegzahnschlitzen (4041) sind und zu zwischen den Einwegzahnschlitzen (4041) angeordneten Kerben formkomplementär sind, wobei, wenn sich der Anti-Rückrotationsbefestiger (405) distal bewegt, die elastischen Anti-Rückrotationsklingen (4051) sich wiederholt verformen und über die Einwegzahnschlitze (4041) gleiten, wodurch eine flüssige Bewegung der Außenhülle (100) ermöglicht ist, und wobei jede mögliche proximale Bewegung des Anti-Rückrotationsbefestigers (405) aufgrund einer begrenzenden Wirkung der Einwegzahnschlitze (4041) auf die Anti-Rückrotationsklingen (4051) verhindert ist, um zu verhindern, dass die Außenhülle (100) sich proximal bewegt.

4. Griffanordnung (400) nach Anspruch 3, wobei der Anti-Rückrotationsbefestiger (405) ferner ein Verbindungsbestandteil (4052) umfasst, wobei die elastischen Anti-Rückrotationsklingen (4051) an dem Verbindungsbestandteil (4052) angeordnet sind, und wobei das Verbindungsbestandteil (4052) eine ringförmige Struktur ist, die über dem Außenhüllenverbinder (402) angebracht ist.

5. Griffanordnung (400) nach Anspruch 4, wobei die ringförmige Struktur eine Kerbe (4053) definiert, die sich parallel zu einer axialen Richtung der Außenhülle (100) erstreckt, wobei ein I-förmiger Vorsprung (4021) von einer Oberfläche des Außenhüllenverbinders (402) hervorsteht und ein vertikales Teil umfasst, das parallel zur axialen Richtung der Außenhülle (100) orientiert ist, und wobei die ringförmige Struktur über dem Außenhüllenverbinder (402) mit Anliegen von gegenüberliegenden Kanten der Kerbe (4053) an gegenüberliegenden Flächen vertikaler Teile des I-förmigen Vorsprungs (4021) montiert ist und dadurch an dem Außenhüllenverbinder (402) befestigt ist.

6. Griffanordnung (400) nach Anspruch 4, wobei die ringförmige Struktur eine L-förmige Kerbe (4054) definiert, die einen Längsabschnitt umfasst, der sich parallel zu einer axialen Richtung der Außenhülle (100) erstreckt, und einen Querabschnitt, der sich senkrecht zu der axialen Richtung der Außenhülle (100) erstreckt, wobei ein Vorsprung (4022) von einer Oberfläche des Außenhüllenverbinders (402) vorsteht, und wobei eine ringförmige Struktur über den Außenhüllenverbinder (402) durch Vorschieben des Vorsprungs (4022) innerhalb des Längsabschnitts der L-förmigen Kerbe (4054) entlang der axialen Richtung der Außenhülle, bis der Vorsprung (4022) die gegenüberliegende Endwand des Längsabschnitts erreicht, und anschließendes Rotieren der ringförmigen Struktur, so dass der Vorsprung (4022) in Kontakt mit der gegenüberliegenden Endwand des Querabschnitts kommt, und dadurch in der Verschiebung in der axialen Richtung der Außenhülle (100) und in der Rotation in einer Umfangsrichtung begrenzt ist, montiert ist.

7. Griffanordnung (400) nach einem der Ansprüche 1 bis 6, ferner umfassend ein vorderes Griffteil (406), das mit dem hinteren Griffteil (401) entlang einer axialen Richtung der Außenhülle (100) mit einem Verriegelungsbestandteil (4061) gekoppelt ist, das eine relative Bewegung zwischen dem Schieber (403) und dem hintere Griffteil (401) ermöglicht oder verhindert.

8. Griffanordnung (400) nach Anspruch 7, wobei das Verriegelungsbestandteil (4061) ein Schalter ist, der an einem Ende des vorderen Griffteils (406) proximal zum hinteren Griffteil (401) angeordnet ist, und wobei, wenn der Schalter in Richtung des hinteren Griffteils (401) gedrückt wird, ein Teil des Schalters in einen entsprechenden Verriegelungsslot im hinteren Griffteil (401) eingeführt ist, wodurch eine Rotation des hinteren Griffteils (401) relativ zum vorderen Griffteil (406) begrenzt ist, und somit eine relative Bewegung zwischen dem Schieber (403) und dem hinteren Griffteil (401) verhindert ist.

9. Griffanordnung (400) nach Anspruch 1, wobei eine Transplantathülse (301) zwischen dem Stent und der Außenhülle (100) vorgesehen ist, und wobei die Griffanordnung ferner eine erste Nase (410) in axialer Verbindung mit der Transplantathülse (301) über einen Transplantateinführungsführungsdraht (303) umfasst.

10. Griffanordnung (400) nach Anspruch 9, wobei der Stent ein proximales Ende aufweist, das durch eine mehrstufige Einführungsanordnung (200) gehalten wird, wobei die Griffanordnung (400) ferner eine zweite Nase (411) in axialer Verbindung mit der mehrstufigen Einführungsanordnung (200) über mehrstufige Einführungsführungsdrähte (205) umfasst, und wobei die mehrstufige Einführungsanordnung (200) eine konische Spitze (201), einen mehrstufigen Einführungsanker (202), eine mehrstufige Einführungsbasis (203), eine Vielzahl von mehrstufigen Einführungsstangen (204) und mehrstufige Einführungsführungsdrähte (205) umfasst, wobei die konische Spitze (201) ein distales Ende aufweist, das eine Vielzahl von Befestigungsbohrungen (2011) zur Aufnahme der entsprechenden mehrstufigen Einführungsstangen (204) definiert, wobei der mehrstufige Einführungsanker (202) eine Vielzahl von Führungsbohrungen (2021) definiert, die den Durchgang der entsprechenden mehrstufigen Einführungsstangen (204) ermöglichen, wobei die Vielzahl von mehrstufigen Einführungsstangen (204) jeweils ein distales Ende in fester Verbindung mit der mehrstufigen Einführungsbasis (203) und ein proximales Ende aufweisen, das durch eine entsprechende der Führungsbohrungen (2021) des mehrstufigen Einführungsankers (202) hindurchgeht und in einer entsprechenden der Befestigungsbohrungen (2011) der konischen Spitze (201) aufgenommen wird, wobei die mehrstufigen Einführungsführungsdrähte (205) jeweils ein proximales Ende in fester Verbindung mit der mehrstufigen Einführungsbasis (203) aufweisen, wobei die mehrstufigen Einführungsstangen (204) Segmente zwischen der konischen Spitze (201) und dem mehrstufigen Einführungsanker (202) aufweisen, die ausgebildet sind, durch ein proximales Ende eines Stents eingeführt zu werden und dieses dadurch zu halten.

11. Griffanordnung (400) nach Anspruch 10, ferner umfassend einen Griffknopf (407), eine Steuerscheibe (408) und eine Endabdeckung (409), wobei der Griffknopf (407) in Verbindung mit einem distalen Ende des hinteren Griffteils (401) ist, wobei die Steuerscheibe (408) eine ringförmige Struktur ist, die über und in rotierbarer Verbindung mit dem Griffknopf (407) montiert ist, wobei die Endabdeckung (409) an einem distalen Ende des Griffknopfs (407) angeordnet ist und ausgebildet ist, eine Bewegung der Steuerscheibe (408) entlang einer axialen Richtung der Außenhülle (100) zu begrenzen.

12. Griffanordnung (400) nach Anspruch 11, wobei zwei Nuten (4071) mit halbkreisförmigen Querschnitten, die sich entlang der axialen Richtung der Außenhülle (100) erstrecken, in einem zur Steuerscheibe (408) proximalen Abschnitt des Griffknopfs (407) definiert sind, wobei die ersten und zweiten Nasen (410, 411) entsprechende Nasenschäfte (4101, 4111) umfassen, die durch Löcher in der Endabdeckung (409) hindurchgehen und in den zwei Nuten (4071) aufgenommen werden, wobei die Nasenschäfte (4101, 4111) der ersten und zweiten Nasen (410, 411) jeweilige Begrenzungsvorsprünge (4102, 4112) definieren, die beide von einer Mitte der Steuerscheibe (408) weg hinausragen, wobei ein Innendurchmesser der Steuerscheibe (408) ausgebildet ist, eine Verschiebung der Begrenzungsvorsprünge (4102, 4112) entlang der axialen Richtung der Außenhülle (100) zu begrenzen, so dass eine Verschiebung der Nasenschäfte (4101, 4111) aus den beiden Nuten (4071) vermieden wird, wobei eine in einer Innenfläche der Steuerscheibe (408) ausgebildete Einführungsaussparung (4081) zu den Begrenzungsvorsprüngen (4102, 4112) formkomplementär ist, und wobei, wenn die Einführungsaussparung (4081) in Positionsübereinstimmung mit einer der zwei Nuten (4071) rotiert wird, der eine der Nasenschäfte (4101, 4111), der in der einen der beiden Nuten (4071) aufgenommen ist, von derselben abziehbar ist.

13. Griffanordnung (400) nach Anspruch 11 oder 12, wobei Einwegzähne (4082) entlang einem Innenumfang der Steuerscheibe (408) vorgesehen sind, wobei eine elastische Einwegklinge (4077) mit einer Spitze, die formkomplementär zu Kerben zwischen den Einwegzähnen (4082) ist, von einem äußeren Umfang des Griffknopfes (407) vorsteht, und wobei, wenn die Steuerscheibe (408) in einer Richtung rotiert, die elastische Einwegklinge (4077) sich wiederholt verformt und über die Einwegzähne (4082) gleitet, wodurch eine flüssige Rotation der Steuerscheibe (408) ermöglicht wird, und wobei irgendeine mögliche Rotation der Steuerscheibe (408) in einer entgegengesetzten Richtung aufgrund einer Begrenzungswirkung der Einwegzähne (4082) an der elastischen Einwegklinge (4077) verhindert wird, um zu verhindern, dass die Steuerscheibe (408) rotiert.

14. Griffanordnung (400) nach Anspruch 11 oder 12, wobei der Griffknopf (407) eine linke Griffknopfhälfte (4072) und eine rechte Griffknopfhälfte (4073) umfasst, wobei die linke Griffknopfhälfte (4072) zwei linke halbkreisförmige Nuten definiert, die sich entlang der axialen Richtung der Außenhülle (100) erstrecken, wobei die rechte Griffknopfhälfte (4073) an entsprechenden Positionen derselben zwei rechte halbkreisförmige Nuten definiert, die sich entlang der axialen Richtung der Außenhülle (100) erstrecken, wobei die linken halbkreisförmigen Nuten mit radial vorstehenden Montagestiften (4075) versehen sind, wobei die rechten halbkreisförmigen Nuten an entsprechenden Positionen derselben mit sich radial erstreckenden Montagelöchern versehen sind, wobei, wenn die Montagestifte (4075) in die Montagelöcher eingeführt sind, die linke Griffknopfhälfte (4072) mit der rechten Griffkopfhälfte (4073) zusammengebaut ist und die beiden linken halbkreisförmigen Nuten und die beiden rechten halbkreisförmigen Nuten zwei Bohrungen (4074) bilden, durch die der Transplantateinführungsführungsdraht (303) und die mehrstufigen Einführungsführungsdrähte (205) entsprechend eingeführt sind, wobei Teile der Montagestifte (4075) in den Bohrungen (4074) verbleiben und verhindern, dass irgendwelche der Führungsdrähte in einem Spalt (4076) zwischen der linken Griffknopfhälfte (4072) und der rechten Griffknopfhälfte (4073) stecken bleiben.

15. Griffanordnung (400) nach Anspruch 11 oder 12, wobei die ersten und zweiten Nasen (410, 411) ferner jeweilige Nasengriffe (4103, 4113) und jeweilige Begrenzungselemente (4104, 4114) umfassen, wobei die Nasengriffe (4103 , 4113) mit den entsprechenden Nasenschäften (4101, 4111) verbunden sind, die durch die entsprechenden Begrenzungselemente (4104 , 4114) eingeführt werden, wobei das Begrenzungselement (4104 , 4114) der ersten Nase (410) ausgebildet ist, die distale Bewegung der Begrenzungselement (4104, 4114) der zweiten Nase (411) zu begrenzen.

16. Griffanordnung (400) nach Anspruch 15, wobei eine partielle Überlappung zwischen Vorsprüngen der Begrenzungselemente (4104, 4114) der ersten und zweiten Nase (410, 411) entlang der axialen Richtung der Außenhülle (100) vorhanden ist, und wobei ein Abschnitt des Begrenzungselements (4104, 4114) der zweiten Nase (411), der zur Überlappung beiträgt, proximal in Bezug auf einen Abschnitt des Begrenzungselements (4104, 4114) der ersten Nase (410) angeordnet ist, der zur Überlappung beiträgt.

17. Stenteinführungssystem, umfassend eine Außenhülle (100), eine mehrstufige Einführungsanordnung (200), eine Transplantathülsenanordnung (300) und die Griffanordnung (400) nach einem der Ansprüche 1 bis 16, wobei die Transplantathülsenanordnung (300) eine Transplantathülse (301) umfasst, in der ein Stent gecrimpt und aufgenommen wird, wobei der Stent ein proximales Ende aufweist, das durch die mehrstufige Einführungsanordnung (200), die innerhalb der Außenhülle (100) angeordnete Transplantathülse (301), die Griffanordnung (400) in Verbindung mit jeder der Außenhülle (100), der Transplantathülsenanordnung (300) und der mehrstufigen Einführungsanordnung (200) gehalten wird,
wobei die mehrstufige Einführungsanordnung (200) eine konische Spitze (201), einen mehrstufigen Einführungsanker (202), eine mehrstufige Einführungsbasis (203), eine Vielzahl von mehrstufigen Einführungsstangen (204) und mehrstufige Einführungsführungsdrähte (205) umfasst, wobei die konische Spitze (201) ein distales Ende aufweist, das eine Vielzahl von Befestigungsbohrungen (2011) zur Aufnahme der entsprechenden mehrstufigen Einführungsstangen (204) definiert, wobei der mehrstufige Einführungsanker (202) eine Vielzahl von Führungsbohrungen (2021) definiert, die den Durchgang der entsprechenden mehrstufigen Einführungsstangen (204) durch dieselben ermöglichen, wobei die Vielzahl von mehrstufigen Einführungsstangen (204) jeweils ein distales Ende in fester Verbindung mit der mehrstufigen Einführungsbasis (203) und ein proximales Ende aufweisen, das durch eine entsprechende der Führungsbohrungen (2021) des mehrstufig Einführungsankers (202) verläuft und in einer entsprechenden der Befestigungsbohrungen (2011) der konischen Spitze (201) aufgenommen wird, wobei die mehrstufigen Einführungsführungsdrähte (205) jeweils ein proximales Ende in fester Verbindung mit der mehrstufigen Einführungsbasis (203) aufweisen, wobei die mehrstufigen Einführungsstangen (204) Segmente zwischen der konischen Spitze (201) und dem mehrstufigen Einführungsanker (202) aufweisen, die ausgebildet sind, durch ein proximales Ende eines Stents eingeführt zu werden und dieses dadurch zu halten.

## Revendications

1. Ensemble de poignée (400) d'un système de pose d'endoprothèse, comprenant une partie de poignée arrière (401), un coulisseau (403) et deux tiges de guidage (404), la partie de poignée arrière (401) étant en connexion filetée avec le coulisseau (403), le coulisseau (403) en connexion coulissante avec les deux tiges de guidage (404), les deux tiges de guidage (404) étant disposées parallèlement à une gaine externe (100) pour recevoir une endoprothèse, et les deux tiges de guidage (404) étant symétriques l'une de l'autre par rapport à un axe du coulisseau (403), le coulisseau (403) en connexion axiale avec la gaine externe (100), et le coulisseau (403) étant muni d'un filetage externe sur une surface externe de celui-ci, qui vient en prise dans un filetage interne sur une surface interne de la partie de poignée arrière (401), et lorsque la partie de poignée arrière (401) est tournée, le coulisseau (403) se déplaçant le long des deux tiges de guidage (404) et provoquant un mouvement axial de la gaine externe (100),
la gaine externe (100) comprenant une couche interne (101), une couche de renforcement (102) et une couche externe (103) qui sont agencées de l'intérieur vers l'extérieur, la couche de renforcement (102) comprenant au moins un premier élément de renforcement (1021) et au moins un second élément de renforcement (1022), le premier élément de renforcement (1021) s'enroulant en spirale le long d'un axe de la gaine externe (100), le second élément de renforcement (1022) s'étendant parallèlement à l'axe de la gaine externe (100).

2. Ensemble de poignée (400) selon la revendication 1, comprenant en outre un raccord de gaine externe (402) et une fixation anti-rotation inverse (405), le raccord de gaine externe (402) étant en connexion avec la gaine externe (100) et le coulisseau (403) à ses extrémités respectives, la fixation anti-rotation inverse (405) étant disposée sur le raccord de gaine externe (402) et étant mobile de manière unidirectionnelle le long des deux tiges de guidage (404).

3. Ensemble de poignée (400) selon la revendication 2, l'attache anti-rotation inverse (405) comprenant des lames élastiques anti-rotation inverse (4051), des fentes à dents unidirectionnelles (4041) étant prévues dans des parties des deux tiges de guidage (404) proximales aux lames élastiques anti-rotation inverse (4051), les lames élastiques anti-rotation inverse (4051) ayant des pointes qui sont proximales aux fentes à dents unidirectionnelles (4041) et de forme complémentaire aux encoches entre les fentes à dents unidirectionnelles (4041), lorsque la fixation anti-rotation inverse (405) se déplace de manière distale, les lames élastiques anti-rotation inverse (4051) se déformant et glissant de manière répétée sur les fentes à dents unidirectionnelles (4041), permettant un déplacement en douceur de la gaine externe (100), et tout mouvement proximal possible de la fixation anti-rotation inverse (405) étant empêché en raison d'un effet de limitation des fentes à dents unidirectionnelles (4041) sur les lames élastiques anti-rotation inverse (4051) pour empêcher la gaine externe (100) de se déplacer de manière proximale.

4. Ensemble de poignée (400) selon la revendication 3, la fixation anti-rotation inverse (405) comprenant en outre une caractéristique de connexion (4052), les lames élastiques anti-rotation inverse (4051) disposées sur la caractéristique de connexion (4052), et la caractéristique de connexion (4052) étant une structure annulaire ajustée sur le raccord de gaine externe (402).

5. Ensemble de poignée (400) selon la revendication 4, la structure annulaire définissant une encoche (4053) s'étendant parallèlement à une direction axiale de la gaine externe (100), une saillie en forme de I (4021) faisant saillie depuis une surface du raccord de gaine externe (402) et comprenant une pièce verticale orientée parallèlement à la direction axiale de la gaine externe (100), et la structure annulaire étant ajustée sur et ainsi fixée au raccord de gaine externe (402) avec les bords opposés de l'encoche (4053) venant en butée contre les faces opposées de la pièce verticale de la saillie en forme de I (4021).

6. Ensemble de poignée (400) selon la revendication 4, la structure annulaire définissant une encoche en forme de L (4054) comprenant une partie longitudinale s'étendant parallèlement à une direction axiale de la gaine externe (100) et une partie transversale s'étendant perpendiculairement à la direction axiale de la gaine externe (100), une saillie (4022) faisant saillie depuis une surface du raccord de gaine externe (402), et une structure annulaire étant ajustée sur le raccord de gaine externe (402) en faisant avancer la saillie (4022) à l'intérieur de la partie longitudinale de l'encoche en forme de L (4054) le long de la direction axiale de la gaine externe jusqu'à ce que la saillie (4022) atteigne la paroi d'extrémité opposée de la partie longitudinale, puis en faisant tourner la structure annulaire de sorte que la saillie (4022) vienne en contact avec la paroi d'extrémité opposée de la partie transversale et soit ainsi limitée en translation dans la direction axiale de la gaine externe (100) et en rotation dans une direction circonférentielle.

7. Ensemble de poignée (400) selon l'une quelconque des revendications 1 à 6, comprenant en outre une partie de poignée avant (406) couplée à la partie de poignée arrière (401) le long d'une direction axiale de la gaine externe (100), avec une caractéristique de verrouillage (4061) permettant ou empêchant un mouvement relatif entre le coulisseau (403) et la partie de poignée arrière (401).

8. Ensemble de poignée (400) selon la revendication 7, la caractéristique de verrouillage (4061) étant un commutateur disposé sur une extrémité de la partie de poignée avant (406) proximale à la partie de poignée arrière (401), et lorsque le commutateur est poussé vers la partie de poignée arrière (401), une partie du commutateur est insérée dans une fente de verrouillage correspondante dans la partie de poignée arrière (401), limitant ainsi la rotation de la partie de poignée arrière (401) par rapport à la partie de poignée avant (406) et empêchant ainsi un mouvement relatif entre le coulisseau (403) et la partie de poignée arrière (401).

9. Ensemble de poignée (400) selon la revendication 1, un manchon de greffe (301) étant prévu entre l'endoprothèse et la gaine externe (100), et l'ensemble de poignée comprenant en outre une première patte (410) en connexion axiale avec le manchon de greffe (301) par l'intermédiaire d'un fil de guidage de déploiement de greffe (303).

10. Ensemble de poignée (400) selon la revendication 9, l'endoprothèse ayant une extrémité proximale contrainte par un ensemble de déploiement multi-étages (200), l'ensemble de poignée (400) comprenant en outre une seconde patte (411) en connexion axiale avec l'ensemble de déploiement multi-étages (200) par l'intermédiaire des fils de guidage de déploiement multi-étages (205), et l'ensemble de déploiement multi-étages (200) comprenant une pointe conique (201), un ancrage de déploiement multi-étages (202), une base de déploiement multi-étages (203), une pluralité de tiges de déploiement multi-étages (204) et des fils de guidage de déploiement multi-étages (205), la pointe conique (201) ayant une extrémité distale définissant une pluralité d'alésages de fixation (2011) pour recevoir les tiges de déploiement multi-étages correspondantes (204), l'ancrage de déploiement multi-étages (202) définissant une pluralité de trous de guidage (2021) permettant le passage à travers eux des tiges de déploiement multi-étages correspondantes (204), la pluralité de tiges de déploiement multi-étages (204) ayant chacune une extrémité distale en connexion fixe avec la base de déploiement multi-étages (203) et une extrémité proximale qui passe à travers un trou correspondant des trous de guidage (2021) de l'ancrage de déploiement multi-étages (202) et reçue dans un trou correspondant des trous de fixation (2011) de la pointe conique (201), les fils de guidage de déploiement multi-étages (205) ayant chacun une extrémité proximale en connexion fixe avec la base de déploiement multi-étages (203), les tiges de déploiement multi-étages (204) ayant des segments entre la pointe conique (201) et l'ancrage de déploiement multi-étages (202), qui sont configurés pour être insérés à travers et ainsi contraindre une extrémité proximale d'une endoprothèse.

11. Ensemble de poignée (400) selon la revendication 10, comprenant en outre un bouton de poignée (407), un disque de commande (408) et un couvercle d'extrémité (409), le bouton de poignée (407) en connexion avec une extrémité distale de la partie de poignée arrière (401), le disque de commande (408) étant une structure annulaire ajustée sur et en connexion rotative avec le bouton de poignée (407), le couvercle d'extrémité (409) disposé à une extrémité distale du bouton de poignée (407) et configuré pour limiter un mouvement du disque de commande (408) le long d'une direction axiale de la gaine externe (100).

12. Ensemble de poignée (400) selon la revendication 11, deux rainures (4071) ayant des sections transversales semi-circulaires, qui s'étendent le long de la direction axiale de la gaine externe (100), étant définies dans une partie du bouton de poignée (407) proximale au disque de commande (408), les première et seconde pattes (410, 411) comprenant des arbres de patte respectifs (4101, 4111) qui passent à travers des trous dans le couvercle d'extrémité (409) et sont reçus dans les deux rainures (4071), les arbres de patte (4101, 4111) des première et seconde pattes (410, 411) définissant des saillies de limitation respectives (4102, 4112) faisant toutes deux saillie à partir d'un centre du disque de commande (408), un diamètre intérieur du disque de commande (408) étant conçu pour limiter la translation des saillies de limitation (4102, 4112) le long de la direction axiale de la gaine externe (100) de manière à éviter le délogement des arbres de pattes (4101, 4111) des deux rainures (4071), un évidement de déploiement (4081) formé dans une surface interne du disque de commande (408) étant de forme complémentaire aux saillies de limitation (4102, 4112), et lorsque l'évidement de déploiement (4081) est tourné en coïncidence de position avec l'une des deux rainures (4071), l'un des arbres de patte (4101, 4111) reçu dans l'une des deux rainures (4071) peut être retiré de celle-ci.

13. Ensemble de poignée (400) selon la revendication 11 ou 12, des dents unidirectionnelles (4082) étant prévues le long d'une circonférence interne du disque de commande (408), une lame élastique unidirectionnelle (4077) avec une pointe qui est de forme complémentaire aux encoches entre les dents unidirectionnelles (4082) faisant saillie à partir d'une circonférence externe du bouton de poignée (407), et lorsque le disque de commande (408) tourne dans une direction, la lame élastique unidirectionnelle (4077) se déformant et glissant de manière répétée sur les dents unidirectionnelles (4082), permettant une rotation régulière du disque de commande (408), et toute rotation possible du disque de commande (408) dans une direction opposée étant empêchée en raison d'un effet de limitation des dents unidirectionnelles (4082) sur la lame élastique unidirectionnelle (4077) pour empêcher le disque de commande (408) de tourner.

14. Ensemble de poignée (400) selon la revendication 11 ou 12, le bouton de poignée (407) comprenant une moitié de bouton de poignée gauche (4072) et une moitié de bouton de poignée droite (4073), la moitié de bouton de poignée gauche (4072) définissant deux rainures semi-circulaires gauches s'étendant le long de la direction axiale de la gaine externe (100), la moitié de bouton de poignée droite (4073), à des positions correspondantes de celle-ci, définissant deux rainures semi-circulaires droites s'étendant le long de la direction axiale de la gaine externe (100), les rainures semi-circulaires gauches étant pourvues de broches d'assemblage faisant saillie radialement (4075), les rainures semi-circulaires droites, à des positions correspondantes de celles-ci, munies de trous d'assemblage s'étendant radialement, lorsque les broches d'assemblage (4075) sont insérées dans les trous d'assemblage, la moitié de bouton de poignée gauche (4072) étant assemblée avec la moitié de bouton de poignée droite (4073) et les deux rainures semi-circulaires gauches et les deux rainures semi-circulaires droites formant deux alésages (4074) à travers lesquels le fil de guidage de déploiement de greffe (303) et les fils de guidage de déploiement multi-étages (205) sont insérés de manière correspondante, des parties des broches d'assemblage (4075) restant dans les alésages (4074) pour empêcher que l'un quelconque des fils de guidage ne soit coincé dans un espace (4076) entre la moitié de bouton de poignée gauche (4072) et la moitié de bouton de poignée droite (4073).

15. Ensemble de poignée (400) selon la revendication 11 ou 12, les première et seconde pattes (410, 411) comprenant en outre des poignées de patte respectives (4103, 4113) et des éléments de limitation respectifs (4104, 4114), les poignées de patte (4103, 4113) étant connectées aux arbres de patte correspondants (4101, 4111) qui sont insérés à travers les éléments de limitation correspondants (4104, 4114), l'élément de limitation (4104, 4114) de la première patte (410) étant configuré pour limiter le mouvement distal de l'élément de limitation (4104, 4114) de la seconde patte (411).

16. Ensemble de poignée (400) selon la revendication 15, un chevauchement partiel ayant lieu entre les projections des éléments de limitation (4104, 4114) des première et seconde pattes (410, 411) le long de la direction axiale de la gaine externe (100), et une partie de l'élément de limitation (4104, 4114) de la seconde patte (411) qui contribue au chevauchement étant disposée de manière proximale par rapport à une partie de l'élément de limitation (4104, 4114) de la première patte (410) qui contribue au chevauchement.

17. Système de mise en place d'une endoprothèse, comprenant une gaine externe (100), un ensemble de déploiement multi-étages (200), un ensemble de manchon de greffe (300) et l'ensemble de poignée (400) selon l'une quelconque des revendications 1 à 16, l'ensemble de manchon de greffe (300) comprenant un manchon de greffe (301) dans lequel une endoprothèse est sertie et reçue, l'endoprothèse ayant une extrémité proximale contrainte par l'ensemble de déploiement multi-étages (200), le manchon de greffe (301) disposé à l'intérieur de la gaine externe (100), l'ensemble de poignée (400) en connexion avec chacun de la gaine externe (100), de l'ensemble de manchon de greffe (300) et de l'ensemble de déploiement multi-étages (200),
l'ensemble de déploiement multi-étages (200) comprenant une pointe conique (201), un ancrage de déploiement multi-étages (202), une base de déploiement multi-étages (203), une pluralité de tiges de déploiement multi-étages (204) et des fils de guidage de déploiement multi-étages (205), la pointe conique (201) ayant une extrémité distale définissant une pluralité d'alésages de fixation (2011) pour recevoir les tiges de déploiement multi-étages correspondantes (204), l'ancrage de déploiement multi-étages (202) définissant une pluralité d'alésages de guidage (2021) permettant le passage à travers eux des tiges de déploiement multi-étages correspondantes (204), la pluralité de tiges de déploiement multi-étages (204) ayant chacune une extrémité distale en connexion fixe avec la base de déploiement multi-étages (203) et une extrémité proximale qui passe à travers un alésage correspondant des alésages de guidage (2021) de l'ancrage de déploiement multi-étages (202) et reçue dans un alésage correspondant des alésages de fixation (2011) de la pointe conique (201), les fils de guidage de déploiement multi-étages (205) ayant chacun une extrémité proximale en connexion fixe avec la base de déploiement multi-étages (203), les tiges de déploiement multi-étages (204) ayant des segments entre la pointe conique (201) et l'ancrage de déploiement multi-étages (202), qui sont configurés pour être insérés à travers et ainsi contraindre une extrémité proximale d'une endoprothèse.
